(19) Europäisches Patentamt

European Patent Office

Office européen des brevets

(11) **EP 1 092 782 B1**

(12) **EUROPEAN PATENT SPECIFICATION**

(45) Date of publication and mention
of the grant of the patent:
**08.02.2006 Bulletin 2006/06**

(51) Int Cl.:
*C12Q 1/68* *(2006.01)*    *G01N 27/447* *(2006.01)*

(21) Application number: **00122440.1**

(22) Date of filing: **13.10.2000**

(54) **Genetic screening method and genetic screening apparatus**

Genetisches Screening Verfahren und Vorrichtung

Méthode et appareil de screening génétique

(84) Designated Contracting States:
**DE GB**

(30) Priority: **15.10.1999  JP  29425799**

(43) Date of publication of application:
**18.04.2001  Bulletin 2001/16**

(73) Proprietors:
• **Hitachi, Ltd.**
  **Chiyoda-ku,**
  **Tokyo 101-8010 (JP)**
• **National Cancer Center**
  **Tokyo 104-0045 (JP)**

(72) Inventors:
• **Tomita, Hiroyuki,**
  **c/o Hitachi, Ltd.**
  **Chiyoda-ku,**
  **Tokyo 100-8220 (JP)**
• **Murakawa, Katsuji,**
  **c/o Hitachi, Ltd.**
  **Chiyoda-ku,**
  **Tokyo 100-8220 (JP)**
• **Miyahara, Yuji,**
  **c/o Hitachi, Ltd.**
  **Chiyoda-ku,**
  **Tokyo 100-8220 (JP)**
• **Murakami, Yoshinori,**
  **Nat., Cancer Res., Inst.,**
  **Tokyo 104-0045 (JP)**
• **Sekiya, Takao**
  **Nat., Cancer Res., Inst.,**
  **Tokyo 104-0045 (JP)**

(74) Representative: **Strehl Schübel-Hopf & Partner**
  **Maximilianstrasse 54**
  **80538 München (DE)**

(56) References cited:
• **YOKOZAKI HIROSHI ET AL: "Alterations of p73 preferentially occur in gastric adenocarcinomas with foveolar epithelial phenotype." INTERNATIONAL JOURNAL OF CANCER, vol. 83, no. 2, 8 October 1999 (1999-10-08), pages 192-196, XP002247700 ISSN: 0020-7136**
• **MURAKAMI Y ET AL: "INACTIVATION OF THE RETINOBLASTOMA GENE IN A HUMAN LUNG CARCINOMA CELL LINE DETECTED BY SINGLE-STRAND CONFORMATION POLYMORPHISM ANALYSIS OF THE POLYMERASE CHAIN REACTION PRODUCT OF CDNA" ONCOGENE, BASINGSTOKE, HANTS, GB, vol. 6, no. 1, 1991, pages 37-42, XP000671241 ISSN: 0950-9232**
• **SUGANO K ET AL: "Diagnosis of bladder cancer by analysis of the allelic loss of the p53 gene in urine samples using blunt-end single-strand conformation polymorphism" INTERNATIONAL JOURNAL OF CANCER 1997 UNITED STATES, vol. 74, no. 4, 1997, pages 403-406, XP002247701 ISSN: 0020-7136**
• **SHIGYO MASANORI ET AL: "Allelic loss on chromosome 9 in bladder cancer tissues and urine samples detected by blunt-end single-strand DNA conformation polymorphism." INTERNATIONAL JOURNAL OF CANCER, vol. 78, no. 4, 9 November 1998 (1998-11-09), pages 425-429, XP002247702 ISSN: 0020-7136**
• **MAKINO R ET AL: "F-SSCP: fluorescence-based polymerase chain reaction-single-strand conformation polymorphism (PCR-SSCP) analysis." PCR METHODS AND APPLICATIONS. UNITED STATES AUG 1992, vol. 2, no. 1, August 1992 (1992-08), pages 10-13, XP000644666 ISSN: 1054-9803**

EP 1 092 782 B1

**(Cont. next page)**

• HAYASHI K: "Recent enhancements in SSCP" GENETIC ANALYSIS: BIOMOLECULAR ENGINEERING, ELSEVIER SCIENCE PUBLISHING, US, vol. 14, no. 5-6, February 1999 (1999-02), pages 193-196, XP004158704 ISSN: 1050-3862

**Description**

**Field of the Invention**

The present invention relates to a genetic screening method for the simple and inexpensive detection, as a discrepancy (or imbalance) in the amount of gene expression, of a gene mutation related to cancer and other disorders using nucleic acid (DNA, RNA) extracted from cells from a patient's urine, sputum, feces, swabs, whole blood, plasma, biopsy tissue, bone marrow, pus, wash fluid from the affected area or the like.

**Background of the Invention**

Conventional methods for detecting gene mutation, especially, gene insertion, deletion and the like, include amplifying a gene by polymerase chain reaction, and then detecting the presence or absence of the gene fragments by hybridization (e.g., Southern, Northern hybridization and comparing the size of the fragments. However, in the hybridization method, a sufficient sample amount is required for detection so recently, methods such as the SSCP (Single Strand Conformation Polymorphism) method, the APO (Allele Specific Oligonucleotide) method, the RNase A mismatch cleavage method, the DGGE (Denaturant Gradient Gel Electrophore) method, and the nucleotide sequencing method are widely used.

It is known that the substitution, deletion, or addition of a single nucleotide may be a factor or cause of cancer, and thus detection of a point mutation is required. In the single strand conformation polymorphism (SSCP) method, the change in conformation taken by denatured single stranded DNA fragment in a non-denaturing gel as a result of a single differing nucleotide is detected as a difference in mobility in polyacrylamide electrophoresis (Orita et al., Proc. Natl. Acad. Sci., vol. 86, 2766- 2779 (1989)). In the APO method, mutation is detected by exploiting the inability to form hybrids due to mismatch of a single nucleotide pair (Wallace et al., Nucleic Acid Res., vol. 9, 879- 895 (1981)). With the RNase A mismatch cleavage method, mutation is detected by cleaving an RNA probe with enzyme RNase A at the position at which there occurs an RNA-DNA or RNA-RNA hybrid mismatch (Myers, Nature, vol.313, 495- 498 (1985)). The DGGE method allows for detection of a mutation by exploiting the fact that DNA fragments having a mismatch and DNA fragments not having a mismatch exhibit different degrees of mobility on a denaturant gradient gel (Fischer & Lerman, Cell, vol.16, 191- 200 (1979)).

In the nucleotide sequencing method, the nucleotide sequence of the isolated DNA fragment is directly determined by the deoxy- termination method (Sanger et al, Proc. Natl. Acad. Sci., vol. 7, 5463-5467 (1977)). The amount of obtained information is greatest with the nucleotide sequencing method, but there are problems in that the procedure is complex and time-consuming.

With the SSCP method, the repeatability of results is good, and the presence or absence of a mutation can be rapidly determined. Consequently, this method has become widely employed during recent years. Normally, since the amount of DNA obtainable from a sample is small, the PCR-SSCP method is used, wherein the region to be analyzed is first amplified using PCR and the thus obtained DNA fragments subjected to SSCP analysis (Orita et al, Genomics, vol. 5, 874- 879 (1989)). The PCR-SSCP method has been applied to the genetic diagnosis of digestive organ cancer and bladder cancer (Sugano et al , Int. J. Cancer, vol. 74, 403- 406 (1997)) . In recent years, the RT-PCR-SSCP method which involves extracting mRNA from a sample, obtaining complementary DNA (cDNA) from the mRNA by reverse transcriptase reaction and thereafter conducting PCR-SSCP, has been used as a means for determining the presence or absence of gene expression and quantifying the amount of expression (Murakami et al, Oncogene, vol. 6, 37-42 (1991)).

It should be noted that amount of gene expression is defined as the number of a mRNA involved in total RNA or poly(A) RNA, per unit mass, for example, per $1\mu$g of total RNA or poly(A) RNA. If is possible to isolate DNA having a mutation in the nucleotide sequence of a genomic DNA gene by PCR-SSCP. On the other hand, it is possible to detect a mutation in a gene's nucleotide sequence and gene expression disorder by the RT-PCR-SSCP method using cDNA obtained from mRNA. A gene expression disorder may be caused by, for example, a mutation in the nucleotide sequence of the expression control region upstream of the gene, or a methylation abnormality, or the like. It is possible to gain information using the RT-PCR-SSCP method that cannot be obtained by the PCR-SSCP method.

A sufficient amount of DNA can be amplified by PCR, RT-PCR-SSCP from a small sample amount. However, it is well known that the amplification efficiency of PCR varies frequently according to differences in the reaction instrument-types and heat-resistant DNA polymerase. When $0 \leq r \leq 1$ and n is the number of PCR cycles, the amplification rate C with PCR is described as $C=(1+r)^n$. Theoretically r=1, however the value of r varies frequently according to various factors such as the thermal profile of the PCR reaction, properties of the DNA polymerase (e.g., reproducibility of the enzyme), DNA sequence length, primer sequence, ratio of primer concentration and DNA concentration, and the like. Depending on the circumstances, r may take a value of between 0.6 and 0.8, and since n is generally a value of around 30, the slightest difference in the value of r may result in the value C of PCR being several to tens of times different.

Further, it is known that the efficiency of the production of cDNA from mRNA through reverse transcriptase reaction will vary depending on the type of reverse transcriptase, reaction temperature, template RNA sequence and the like.

As methods of quantitative analysis using PCR, competitive PCR (Gilliard et al, Proc. Natl. Acad. Sci., Vol. 87, 2725- 2729, 1990), kinetic PCR (Wang et al, Proc. Nat1. Acad. Sci., Vol . 86, 9717-9721, 1989), TaqMan PCR (Gelfand et a1, USP5210015 (1993)) have been developed. In competitive PCR, to measure the amount of DNA, DNA of a known concentration is used as an internal standard, and the internal standard DNA is systematically diluted, and then added, and amplified at the same time as the sample DNA. The PCR products are separated by gel electrophoresis, and the number of copies of sample DNA and internal standard DNA are compared using ethidium bromide staining technique. The precision in quantifying the amount of DNA increases with the number of dilutions. However, there is the problem that the amount of sample required and the amount of work increases in proportion to the number of dilutions. In kinetic PCR, PCR is stopped at the logarithmic amplification phase. In the logarithmic amplification phase, the number of PCR amplification products is thought to correlate to the number of sample DNA. Firstly, a plurality of DNA of known concentration are amplified using PCR, and the amounts of obtained PCR amplification product are plotted and a calibration curve (a straight line) is created. Under the same conditions used when creating the calibration curve, DNA of unknown concentration is amplified using PCR and the amount of sample DNA is quantified using the calibration curve. Since in kinetic PCR, the number of PCR cycles is reduced so that the PCR does not saturate, there is the problem that the final concentration of PCR amplification product is low. However, through the use of a fluorescence-labeled primer and laser fluorescence DNA sequencer, slight amounts of PCR amplification products can be detected. TaqMan PCR is a method combining kinetic PCR and a fluorescence-labeled primer by which the amount of amplification product for each cycle can be monitored during the PCR amplification. In the TaqMan PCR method, a calibration curve can simultaneously be created, and there is no need to prepare a calibration curve beforehand. However, enzymes such as heat-resistant polymerase are costly, and since a apparatus is used where optical fiber is introduced into each reaction chamber of the PCR reaction, there is the problem that running costs are high.

Competitive PCR, kinetic PCR, TaqMan PCR are excellent methods. However, it is necessary to firstly prepare a plurality of DNA samples of known concentration as internal standards. The preparation of several internal standard DNA for each sample leads to increased cost and work at the time of examination. In competitive PCR, negative PCR and TaqMan PCR, there is variance in efficiency of PCR amplification due to difference in the type and product of the heat-resistant DNA polymerase, and a margin of error in the amount of gene expression sought. According to simulations, with competitive PCR a margin of error ranging from 7% to 300% may arise (Raeyaekers, Anal. Biochem., vol. 214, 582- 585 (1993)). Generally, with conventional methods of competitive PCR, there is considered to be a dispersion of 30%-50% in the measurement result for amount of gene expression.

Further, with an individual (carrier) having reproductive cellular mutation in one of the two alleles of a cancer suppressive gene or DNA repairing enzyme gene, the frequency of tumor occurrence increases and onset of disease increases somewhat, and an increase in multiple primary cancer and multiple cancer is known, so there is a need for increased efficiency in detection. In order to resolve this problem with the conventional techniques, it is an object of the present invention to provide a genetic screening method suitable for automation and having superior quantification and reproducibility, and a genetic screening apparatus.

**Summary of the invention**

Autosomes of normal human cells are diploid, and there are two alleles derived from father and mother. Where the two alleles have differing sequences and are polymorphic, the gene is said to be a heterozygote. The two alleles can be distinguished by this polymorphism. In cancer cells, where all or part of a chromosome is deleted, and one allele deriving from either the father or mother has been lost, the heterozygosity that can be seen in the DNA of normal cells, cannot be found in cancer cells (Loss of heterozygosity: LOH). Actually, LOH at chromosome sites where tumor- suppressors such as p53 and APC gene are present has been recognized with high frequency in various cancers. The high frequency of cancer is resulted from the inability to suppress cell "canceration" due to the non-existence of the corresponding normal gene. LOH analysis has already been applied in the clarification of the molecular mechanisms of canceration, and in the DNA diagnosis of cancer.

When a nucleotide sequence polymorphism is present in an exon of a gene, mRNAs originating from the two alleles can be distinguished. Messenger RNAs, where genomic imprinting etc. does not arise, are thought to be transcribed from the two alleles in equal amounts. However, due to upstream nucleotide sequence polymorphisms or mutations affecting the control of gene expression, and differences in the 3' terminal sequence of mRNA altering the stability of the mRNA molecule, it is thought that it may cause a "difference in gene expression between alleles . Therefore, it was thought that detection of this "difference in gene expression between alleles" would be a completely new approach to clarifying the physiological and etiological significance of nucleotide sequence polymorphisms and mutations. To date, there have been no attempts to statistically clarify this "difference in gene expression between alleles. " However, with the progress of the Human Genome Project, information regarding nucleotide sequence polymorphisms has been accumulating so that statistical analysis of this "difference in gene expression between alleles" can be validly expected. In particular, proteins of uncertain pathological significance that arise from mis-sense mutations accompanied by amino acid substitutions, have been discovered from analysis of numerous cancers and genetic disorders. The present approach can be expected to contribute to the clarification of mis-sense mutations accompanied by amino acid substitutions.

Further, it is known that where a termination codon occurs in the middle of coding region of a gene as a result of point mutation or frameshift mutation, there is a decrease in mRNA expression. Therefore, when a striking difference in gene expression of mRNA between alleles is found, it is thought that this result reflects sharply etiological conditions including inactivation of the gene, and this phenomenon may be used for a simple method to detect the presence or absence of gene mutations. It is known that there exist various differences in nucleotide sequence between alleles, between individuals, and between populations in human genomic DNA. Since the greater proportion of these sequence differences are not pathological, they are called nucleotide sequence polymorphisms, not mutations. Among nucleotide sequence polymorphisms, single nucleotide polymorphisms (SNP) arising from the substitution of a single nucleotide pair, microsatellite polymorphisms due to differences in the number of repetitions of short repeat sequences of about 2-4 base pairs, and VNTR (Variable Number of Tandem Repeat) polymorphisms that differ in terms of number of repeats and nucleotide sequence in units of several tens of base pairs, are known. Single nucleotide polymorphisms are predicted to exist on average in more than one in every thousand base pairs in human DNA, and have significant value as markers covering the entire human genome. Recently in particular, single nucleotide polymorphisms existing in the exon regions of genes (SNP in cDNA: cSNP) have become the subject of attention as one cause of individual differences in connection with susceptibility to various diseases and sensitivity to pharmaceuticals. In the single strand conformation polymorphism (SSCP) method, a double-stranded DNA fragments amplified by PCR or the like are denatured into single-stranded DNA fragments in the presence of formamide. Thereafter, when separated with non-denatured polyacrylamide gel electrophoresis, since the single-stranded DNA fragments adopt a particular conformation in the nucleotide sequence of each DNA fragment, the complementary single stranded DNA fragments exhibit different mobility each other. Not only the substitution of a single nucleotide, but also the nucleotide deletion or insertion alters the conformation and mobility of the single-stranded DNA fragment, allowing the detection of substitution, deletion and insertion with gel electrophoresis and the separation of abnormal DNA fragments.

Thus, in the method for screening genes according to the present invention, (1) genomic DNA fragments and RNA fragments are obtained from a sample taken from a subject; (2) cDNA fragments are obtained from the said RNA fragments by reverse transcriptase reaction; (3) PCR amplification reaction is conducted using said genomic DNA fragments and said complementary DNA fragments as templates, and a first PCR amplification product derived from the target region of said genomic DNA fragments, and a second PCR amplification product derived from the target region of said complementary DNA fragments, are obtained; (4) the amounts of said first PCR amplification product and of said second PCR amplification product are measured in respect of each allele from which said genomic DNA fragments and said complementary DNA fragments are derived; (5) differences in gene expression between the alleles are detected based on the results of said measurements; and, (6) the presence or absence of genetic abnormality is determined based on the results of said detection.

In other words, the method of the present invention is characterized in that it comprises a first step of obtaining a genomic DNA fragments and RNA fragments from a sample taken from a subject; a second step of obtaining complementary DNA fragments to said RNA fragments by reverse transcriptase reaction; a third step of performing PCR amplification using said genomic DNA fragments and said complementary DNA fragments as templates to obtain a first PCR amplification product derived from the target region of said genomic DNA fragment and a second PCR amplification product derived from the target region of said complementary DNA fragment; a fourth step of measuring the amount of said first PCR amplification product and said second PCR amplification product for each allele from which said genomic DNA fragments and said complementary DNA fragments are derived; a fifth step of detecting the difference in gene expression between the alleles based on the results of said measurements; and, a sixth step of determining the existence or otherwise of genetic abnormality based on said measurement results.

A preferred embodiment of the method of the present invention further comprises a step of blunting the 3' termini of the said first PCR amplification product and the said second PCR amplification product.

Another preferred embodiment of the method of the present invention requires the conditions of the said PCR amplification reaction to be identical in respect of both the said genomic DNA fragment and complementary DNA fragment templates.

In another preferred embodiment of the method of the present invention, said fourth step is conducted by the single strand conformation polymorphism method.

In another preferred embodiment of the method of the present invention, the PCR amplification reaction in said third step uses a fluorescently labeled primer, and the said first PCR amplification product and the said second PCR amplification product obtained in said third step are subjected to electrophoresis, and measurement in said fourth step is conducted by detecting the fluorescence of said fluorescent labels.

In another preferred embodiment of the method of the present invention, each signal intensity of the electrophoretic band of said first PCR amplification product (peak height or peak area) for each of the alleles from which the genomic DNA fragments used as templates are derived, is expressed as "A1 (DNA) " and "A2(DNA)" respectively, and each signal intensity of the electrophoretic band of said second PCR amplification product (peak height or peak area) for each of the alleles from which the complementary DNA fragments used as template are derived, is expressed as "B1 (cDNA) " and "B2 (cDNA) " respectively. Further, the difference in gene expression between the two alleles is detected by comparing a first indicator and a second indicator,

where the first indicator is derived by the following formula:

$$k=A2\,(DNA)\,/A1\,(DNA)$$

or, the following formula:

$$k'=A1\,(DNA)\,/A2\,(DNA)$$

and the second indicator is derived by the following formula:

$$B2\,(cDNA)\,/B1\,(cDNA)$$

or, the following formula:

$$B1\,(cDNA)\,/B2\,(cDNA)\,.$$

In another preferred embodiment of the method of the present invention, the difference in gene expression between alleles is detected by comparing a first indicator and a second indicator; said first indicator and said second indicator being the "difference in gene expression between alleles" and "ratio of gene expression between alleles" respectively; where the "difference in gene expression between alleles" is defined by the following formula:

$$\alpha=|\,(B2\,(cDNA)\,/B1\,(cDNA)\,-A2\,(DNA)\,/A1\,(DNA)\,)\,|$$

or, the following formula:

$$\alpha'=|\,(B1\,(cDNA)\,/B2\,(cDNA)\,-A1\,(DNA)\,/A2\,(DNA)\,)\,|$$

and, the "ratio of gene expression between alleles" is defined by the following formula:

$$(1+(\alpha/k))=(B2\,(cDNA)\,/B1\,(cDNA)\,)\,/(A2\,(DNA)\,/A1\,(DNA)\,)$$

or, the following formula:

$$(1+(\alpha'/k'))=(B1\,(cDNA)\,/B2\,(cDNA)\,)\,/(A1\,(DNA)\,/A2\,(DNA)\,)\,.$$

A further preferred embodiment of the method of the present invention includes a step wherein said first indicator and said second indicator are displayed numerically or graphically.

The apparatus for screening genes according to the present invention comprises a plurality of electrophoresis lanes for electrophoresis of nucleic acid fragments labeled with a fluorescent label; a means for irradiating a laser onto the plurality of electrophoresis lanes; a means for detecting the fluorescence emitted from the fluorescent label by irradiation with a laser; a means for analyzing the electrophoresis pattern of nucleic acid fragments separated by electrophoresis; and a display apparatus for displaying the analysis results.

In a preferred embodiment of the apparatus of the present invention, said display apparatus displays, as either letter(s), numerical value(s), or graph(s), any 1 or more selected from the group consisting of a name of a target gene site, nucleotide sequences of primers, nucleotide sequences of alleles, difference in nucleotide sequence between said alleles, signal intensities of electrophoretic bands of genomic DNA fragments derived from each of said alleles, signal intensities of electrophoretic bands of cDNA fragments derived from each of said alleles, a ratio of signal intensities of electrophoretic bands of genomic DNA fragments derived from each of said alleles, a ratio of signal intensities of electrophoretic bands of cDNA fragments derived from each of the alleles, difference in gene expression between said alleles, a ratio of gene expression between said alleles, and statistically significant difference of said differences and/or ratios of the gene expression between said alleles.

The method of the present invention can be employed as a new screening method for screening a carrier with familial tumor, when peripheral blood lymphocyte is used as a test sample, and as a target, an tumor-suppressor (e.g., p53, BRCA1 or BRCA2) or a DNA fragment which has a polymorphism in an exon of a DNA mismatch repairing enzyme gene (e.g., hMSH2 or hMLH1), are used. This method can therefore contribute greatly to precise examinations. Furthermore, in this method whether there exists abnormality in a specific gene can be rapidly screened with good reproducibility. A subject judged as that there is an abnormality will be subjected to a more precise examination such as determination of gene sequence and identification of mutation. For clinical cases where physiological significance of mutation is not clarified from only identification of the mutation, this method makes it possible to clarify its significance from the point of view of gene expression imbalance. The screening method according to the present invention is not known until now. This method can be utilized for screening mutations of genes associated with onset of cancer and various life-habitual diseases, and for studies on differences between individuals based on extensive nucleotide sequence polymorphisms.

**Brief Description of the Drawings**

Fig. 1 is a flowchart explaining the procedures for screening genes according to the present invention.

Figs. 2 (A) and 2 (B) show a screening example of a normal gene in the screening of a genetic abnormality using a single nucleotide polymorphism according to the method of the present invention.

Figs. 3 (A) and 3 (B) show a screening example of an abnormal gene in the screening of a genetic abnormality using a single nucleotide polymorphism according, to the method of the present invention.

Figs. 4(A) to 4(D) show a screening example applying the method of the present invention to a cancer cell which has no nucleotide sequence polymorphism but mutation in one allele.

Figs. 5(A) and 5(B) show electrophoresis patterns obtained by the method of the present invention. Fig. 5(A) is an electropherogram of a normal sample, and Fig. 5 (B) is an electropherogram of an abnormal sample.

Figs. 6(A) and 6(B) show effect of PCR cycle number in the method of the present invention.

Fig. 7 shows effect of PCR temperature profiles in the method of the present invention.

Fig. 8 shows dispersions of results obtained by three distinct RT-PCRs in the method of the present invention.

Fig. 9 shows dispersions of results obtained for three different samples according to the method of the present invention.

Fig. 10 is a display example that displays screening results obtained in a medical examination of an individual according to the method of the present invention.

Fig. 11 is a display example that displays screening results obtained in a group medical examination according to the method of the present invention.

Fig. 12 is an example of applying probe hybridization to the method of the present invention.

Description of Reference Numerals:

1, paternal allele with single nucleotide polymorphism; 1', maternal allele with single nucleotide polymorphism; 2, site of imbalance (in transcriptional initiation/control region); 3, cancer-specific mutation; 41, electrophoretic band of genomic DNA fragment from allele 1; 42, electrophoretic band of genomic DNA fragment from allele 1'; 43, electrophoretic band of cDNA fragment from allele 1; 44, electrophoretic band of cDNA fragment from allele 1' ; 51, exponential amplification phase; 52, saturation phase; 53, S2(DNA)/S1(DNA) or S2 (cDNA) /S1 (cDNA) ; 54, P2 (DNA) /P1 (DNA) or P2 (cDNA) /P1 (cDNA) ; 110-1, PCR reaction tube; 110-2, PCR reaction tube; 111, PCR buffer; 112, DNA probe that hybridizes specifically with allele 1 of genomic DNA; 113, DNA probe that hybridizes specifically with allele 1' of genomic DNA; 114, genomic DNA fragment; 115, DNA probe that hybridizes specifically with allele 1 of cDNA; 116, DNA probe that hybridizes specifically with allele 1' of cDNA; 117, cDNA fragment.

**Detailed Description of the Invention**

The method for screening genes according to the present invention will be described in detail. It should be noted that DNA fragments amplified using genomic DNA as a template may be referred to as a "genomic DNA fragments", and DNA fragments amplified using complementary DNA as a template may be referred to as "complementary DNA fragments" or "cDNA fragments" in-the specification.

In the first step, DNA fragments and RNA fragments are obtained from a sample taken from a subject. The samples taken from a subject include, but are not limited to, urine, sputum, feces, swabs, whole blood, serum, biopsy tissue, bone marrow, pus, and wash fluid from the affected area. The genomic DNA fragments and the RNA fragments are taken from the same sample. The method for collecting the genomic DNA fragments and the RNA fragments from the sample is not particularly limited and conventional methods are acceptable. Methods for collecting DNA include DNA extraction methods such as the proteinase K-phenol-chloroform method. Further, methods for obtaining amplified DNA by conducting PCR directly using blood or biopsy samples, (Mercier et al, Nucleic Acid Res., vol. 18, 5908, 1990, or Panaccio et al, Nucleic Acid Res., vol. 21, 4656, 1993),can be used. RNA collection methods include, for example, RNA extraction methods such as the guanidine-thiocyanate method. The collected RNA may be either total cellular RNA or poly(A) RNA.

In the second step, complementary DNA fragments of said RNA is obtained through a reverse transcriptase reaction. The reverse transcriptase reaction can be conducted according to conventional techniques. As an enzyme to be used in the reverse transcriptase reaction, enzymes with high optimal reaction temperature such as Superscript II reverse transcriptase or Tth DNA polymerase are preferable, but AMV reverse transcriptase or MoMuLV reverse transcriptase can also be used. As a primer to be used in the reverse transcriptase reaction, any of Oligo dT primer, a random primer of about 6 nucleotides, or a specific primer of about 20-30 nucleotides, or any two or more of these in combination, can be used.

In the third step, PCR is conducted using said genomic DNA fragments and said complementary DNA fragments-as templates. A first PCR amplification product derived from the target region of said genomic DNA fragments, and a second PCR amplification product derived from the target region of said complementary DNA fragments, are obtained. Here, "target region" means the region to be amplified by PCR, and the target region of the genomic DNA fragment and the target region of the complementary DNA fragment are substantially the same region. As a target region any region within the exons of the genomic DNA fragment may be selected, but it is preferable to select a region exhibiting polymorphism. As a region exhibiting polymorphism, for example, a region exhibiting single nucleotide polymorphism (SNP) may be used. The first PCR amplification product derived from the target region of the genomic DNA fragment can be obtained by conducting PCR with the genomic DNA fragment as the template and using a primer able to hybridize with each terminus of the target region. The second PCR amplification product derived from the target region of the complementary DNA fragment can be obtained by conducted PCR with the complementary DNA fragment as a template, and a primer able to hybridize with each terminus of the target region. It is preferable that the primer to be used in PCR is labeled (e.g. with a fluorescent label) in order to conduct detection of the electrophoresis pattern in the fourth step easily and accurately. It is preferably that PCR conditions (e.g. temperatures and durations for denaturation, annealing and extension reaction, PCR cycle number, etc.) are identical when obtaining the "genomic DNA fragment" and the "complementary DNA fragment", and normally PCR is conducted on the same amplification reaction apparatus. It is preferable that the number PCR cycles is as near to identical as possible, but where the template concentrations prior to amplification as between the genomic DNA and complementary DNA clearly differ (e.g. less than one tenth, or greater than ten times), then the number of PCR cycles used when obtaining the "genomic DNA fragment" may differ from the number of PCR used when obtaining the "complementary DNA fragment" by a few cycles. It should be noted that in the third step, amplification methods other than PCR, for example, LCR, NASBA and other such known methods of amplification can be used.

It is preferable to blunt the termini of the PCR amplification product. While this blunting is not essential, it is preferable in terms of improving the precision of the genetic screening method of the present invention. Blunting may be performed, for example, by processing with an enzyme having 3'→5' exonuclease activity such as Klenow fragment.

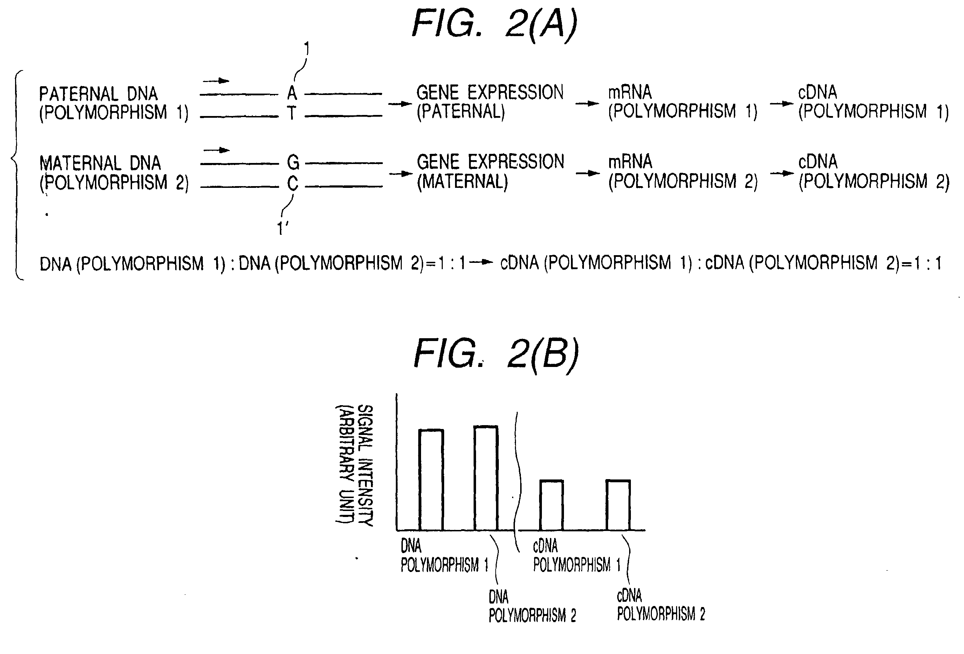

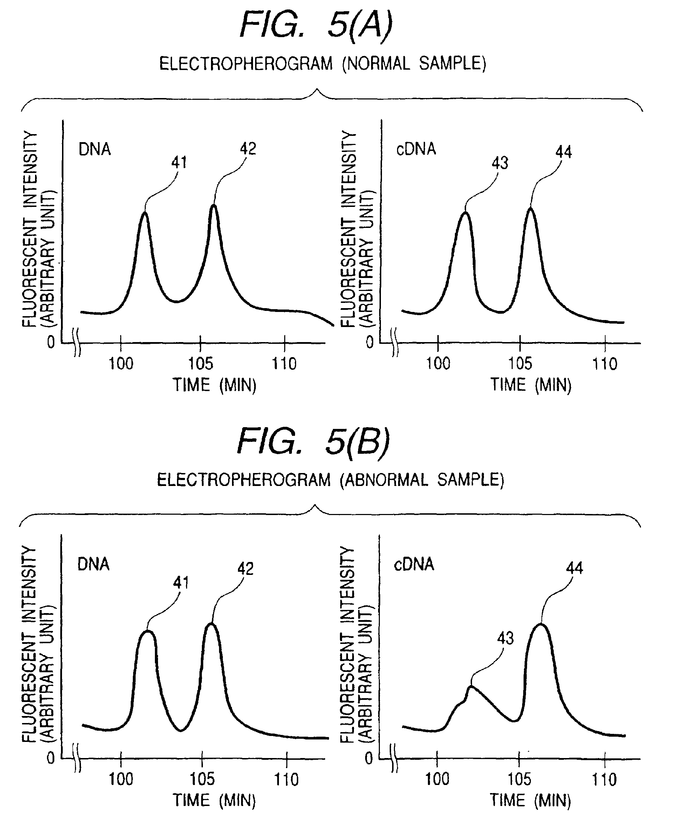

In step 4, the amounts of said first PCR amplification product and said second PCR amplification product are measured in respect of each allele from which said genomic DNA fragment and said complementary DNA fragment are derived. Examples of methods for measuring the amount of PCR amplification product for each allele include a method of measuring based on the electrophoresis pattern obtained by electrophoresis separation of the PCR product, and a method of measuring by probe hybridization using oligonucleotide chips etc. A preferable method of measurement is SSCP. With the SSCP method, a difference in a single nucleotide within the DNA fragment can be detected. Where in the third step a fluorescently labeled primer is used, by subjecting the PCR amplification products to electrophoresis, and detecting fluorescence from the fluorescent label, and by taking electrophoresis time (minutes) as the transverse axis, and fluorescent intensity (relative value) as the vertical axis, an electrophoresis pattern as shown in Figure 5 can be obtained. If the nucleotide sequences of the PCR products differ, their electrophoresis patterns will also differ (Fig. 5). Thus, PCR products derived from the respective alleles of a gene exhibiting heterozygosity, will exhibit differing electrophoresis patterns. Therefore, it is possible thereby to measure for each allele the amount of PCR amplification product based on this electrophoresis pattern. In the analysis of genomic DNA according to the SSCP method shown in Figures 2-4, the electrophoresis patterns of the "genomic DNA fragment" of the paternally derived allele and the maternally derived allele correspond to the electrophoresis patterns of the genomic DNA alleles, whereas the electrophoresis patterns of the "complementary DNA fragment" transcribed from the paternally derived allele and the maternally derived allele correspond to the electrophoresis pattern indicating the expression pattern of RNA.

In the fifth step, the difference in gene expression between alleles is detected based on said measurement results. When the amount of PCR amplification product of the complementary DNA fragment differs for each allele, it is thought that this difference is caused by a difference in gene expression between the alleles. Since the PCR amplification products of the complementary DNA fragments derived from each allele were subject to PCR amplification reaction under the same conditions, then the difference in the amount of PCR amplification product results from a difference in the amount of complementary DNA fragment of a template, that is, a difference in the amount of expressed mRNA between the alleles. In contrast, since it is considered that the amount of genomic DNA fragment of a template is equal between the alleles in normal cells without LOH, it is thought that the amount of PCR amplification product of genomic DNA fragment is equal between the alleles. Therefore, using the ratio of "genomic DNA fragment" between the alleles, the ratio of "complementary DNA fragment" can be accurately calculated for each allele.

In the sixth step, the presence or absence of a genetic abnormality is determined based on said detection results. Where a difference in expression between the alleles is detected, a genetic abnormality is determined to exist. Where no difference in expression between the alleles is detected, no genetic abnormality is determined to exist.

Specifically, where the signal intensity ratio of the electrophoretic band signals for the "genomic DNA fragment" derived from each allele, and the signal intensity ratio of the electrophoretic band signals for the "complementary DNA fragment" derived from each allele - where these ratios differ by more than a given value, it can be determined that there exists a disorder in respect of gene expression of two alleles. Where these ratios do not differ by more than a given value, then it can be determined that exists no disorder in respect of gene expression of two alleles.

Below, the method of the present invention will be explained in detail with reference to figures.

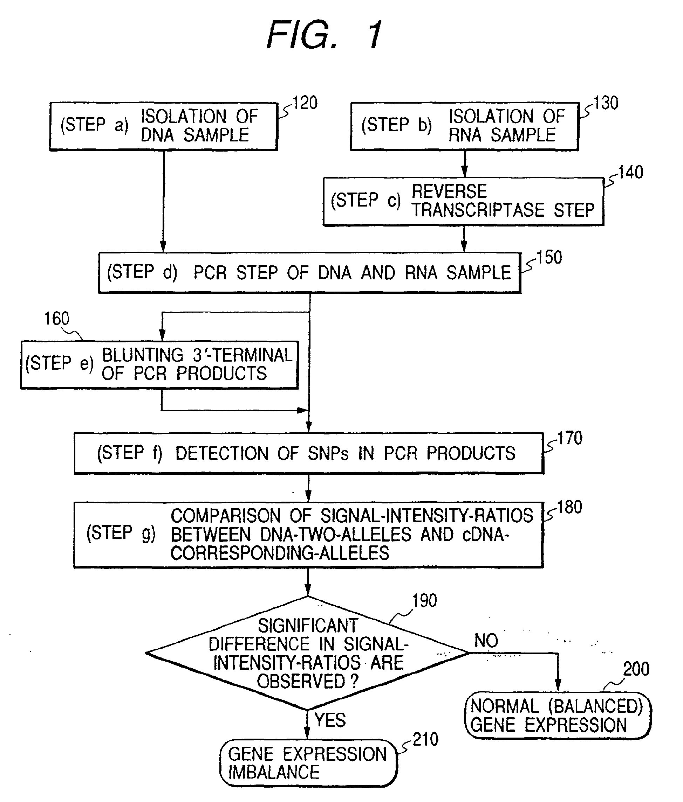

Figure 1 is flowchart indicating an example of the steps of the method of the present invention.

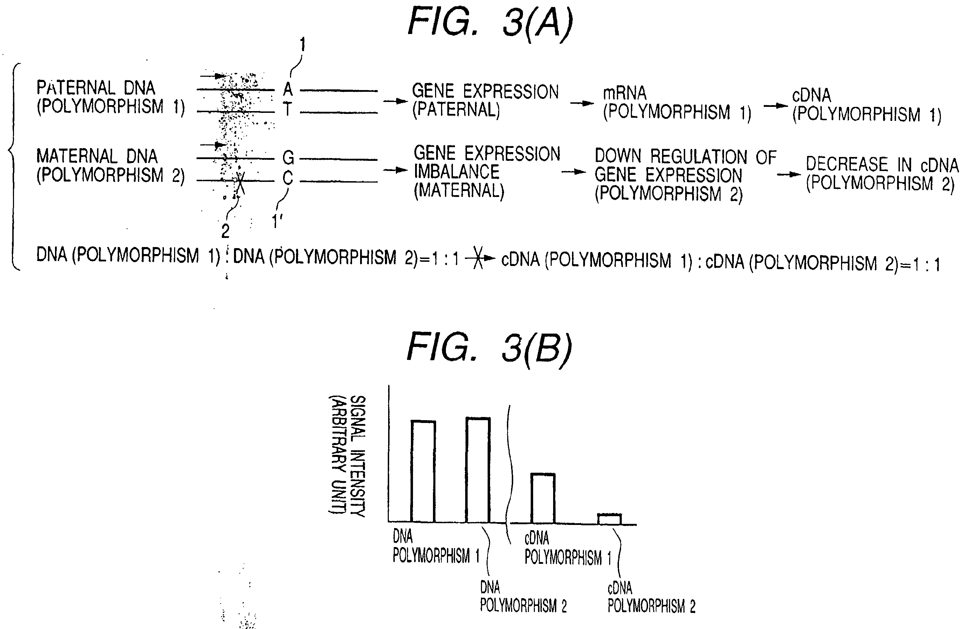

Figures 2 and 3 are figures which explain a screening example where a genetic abnormality is tested using single nucleotide polymorphism. Figure 2 explains a screening example where the gene is normal and Figure 3 explains a screening example where a gene abnormality is suggested.

Figure 2(A) is a figure which explains the relationship between a normal gene and the transcript of the normal gene. Figure 2(B) is a figure indicating the signal intensity of the electrophoretic bands of a "genomic DNA fragment" and a "complementary DNA fragment." Where in the genomic DNA, the DNA fragments of a paternally-derived allele 1 and a maternally-derived allele 1' exhibit single nucleotide polymorphisms, for example as shown in figure 2, where the nucleotide pair of the paternal allele 1 is an AT-pair, and the corresponding nucleotide pair of maternal allele 1' is a GC pair, then since in a normal pair of chromosomes of a normal cell, one chromosome is inherited from each of the father and mother, the ratio between allele 1 and allele 1' is 1:1. In a normal cell these is no loss of heterozygosity (LOH) . If allele 1 and allele 1' express equally, the ratio of mRNA derived from allele 1 and mRNA derived from allele 1' will be 1:1. In the reaction to obtain the "cDNA fragment" derived from allele 1 and the "cDNA fragment" derived from allele 1' , where the reverse transcription reaction and efficiency of PCR are equal, the ratio of allele 1-derived "cDNA fragment" 1 and allele 1' -derived "cDNA fragment" 2 also will be 1:1.

Figure 3(A) is a figure explaining the relationship between a gene where there is a gene expression imbalance suggesting gene abnormality, and the transcription product of the gene. Figure 3(B) is a figure showing the signal intensities of electrophoretic bands of the "genomic DNA fragment" and the "cDNA fragment." As shown in Fig. 3 (A) where there is a mutation 2 in the maternally-derived allele 1' there often occurs a difference in gene expression as between the alleles. Mutation 2 indicates a mutation in the nucleotide sequence of the expression control region upstream of the gene, a mutation arising from a termination codon in the coding region of the gene, an abnormality in the nucleotide sequence of the non-translated region of the mRNA 3'-terminus, or the like.

Generally, for the purpose of diagnosis, it is extremely difficult to sequence, or detect the presence or absence of methylation in respect of, the entire expression control region of a gene, and to establish the significance of genetic mutation. However, regardless of the cause, where the expression of one allele is strikingly lower in comparison to the expression of the other allele, it can be judged that as a result genetic inactivation has occurred. In other word, it can be predicted with that the ratio of allele 1 to allele 1' in respect of the "cDNA fragment", and the ratio of allele 1 to allele 1' in respect of the "genomic DNA fragment". In the case of a normal gene, as shown in Fig. 2 (B), the ratio of allele 1 to allele 1' equals the ratio of the "cDNA fragment" derived from allele 1 to the "cDNA fragment" derived from allele 1'. However, in the case where the presence of a abnormal gene is suggested, as shown in Fig. 3(B), the ratio of allele 1 to allele 1' will not equal the ratio of the "cDNA fragment" derived from allele 1 to the "cDNA fragment" derived from allele 1', thus allowing the detection of gene abnormality as a gene expression imbalance. When determining the ratio of the "cDNA fragment" derived from allele 1 to the "cDNA fragment" derived from allele 1', the use of the conventional RT-PCR-SSCP method can be contemplated. In the conventional RT-PCR-SSCP method, measurement results may vary depending on differences in the reaction apparatus used and difference in DNA polymerase types and articles. Further, there is the possibility that the cell to be examined may have canceration, and LOH occurred. Therefore, where a result indicating that the ratio of the "cDNA fragment" derived from allele 1 to the "cDNA fragment" derived from allele 1' is not 1:1 has been obtained, it is not possible to determine whether this result reflects genetic expression imbalance, or is a result of discrepancies in the PCR reaction, or is due to LOH due to canceration of the cell, or the result is due to a combination of these. In competitive PCR, correlation is made only in respect of the difference due to variation in the PCR reaction. In competitive PCR, DNA in the sample and internal standard DNA fragments of known concentration are, during amplification with an identical set of primers, made to compete and correlation is made of the difference due to reaction conditions.

In the method of the present invention, as shown in Figs. 2 and 3, during amplification, fragments comprising polymorphism-1 and polymorphism-2 respectively are allowed to compete. However, the chain lengths of both DNA fragments, as well as their nucleotide sequences, are identical except for one nucleotide. Therefore, accuracy is similar to, or exceeds that of known competitive PCR methods. With genomic DNA of normal cells in which there is no LOH, the ratio of allele 1 to allele 1' is 1:1. Thus, with the ratio of allele 1 to allele 1' obtained from the result of analysis, as a standard (a ratio of 1:1), the ratio of "cDNA fragments" derived from allele 1 to the "cDNA fragments" derived from allele 1' can be accurately calculated. That is, in respect of the fact that there is no need to prepare internal standard DNA of known concentration, and the two alleles are adopted as an ideal internal standard, the method of the present invention is superior to known competitive PCR methods.

In the electrophoresis pattern, the signal intensity of the electrophoretic band of the "genomic DNA fragment" derive from allele 1 is denoted as A1; the signal intensity of the electrophoretic band of the "genomic DNA fragment derived from allele 1', as A2; the signal intensity of the electrophoretic band of the "complementary DNA fragment" derived from allele 1, as B1; and, the signal intensity of the electrophoretic band of the "complementary DNA fragment" derived from allele 1', as B2. As A1, the peak height P1(DNA) and the peak area P1(DNA); as A2, the peak height P2(DNA) and the peak area P2(DNA); as B1, the peak height P1(cDNA) and the peak area P1(cDNA); and as B2, the peak height P2(cDNA) and the peak area P2 (cDNA), are each used respectively. The ratio of A1 to A2 is denoted as k (Formula I), The difference in the PCR chain extension efficiency, etc., in respect of both alleles is included in k. In normal cells, where the chain extension efficiencies during PCR for both alleles are equal, then k=1 . The value of k is thought to be essentially similar for amplification to obtain "complementary DNA fragments" as well. Thus, if $\alpha$ is defined as the change in signal intensity deriving from the "difference in gene expression between alleles", then B2 can be predicted from Formula II.

$$k = (A2/A1) \quad \ldots \quad (I)$$

$$B2 = B1(\alpha + k) \quad \ldots \quad (II)$$

In the method of the present invention, (A2/A1) is compared with (B2/B1). From the difference between (A2/A1) and (B2/B1), the "difference in gene-expression between alleles" can be calculated (Formula (III)). From the ration of (A2/A1) to (B2/B1), the "gene-expression-imbalance" can be calculated as (1+ ($\alpha$/k)) (Formula (IV))

$$\alpha = | \ (B2/B1) - (A2/A1) | \ \ldots \ (III)$$

$$(1 + (\alpha/k)) = (B2/B1)/(A2/A1) \ \ldots \ (IV)$$

The method of the present invention is unique in that as well as detecting with high precision the "difference in gene expression between alleles" ($\alpha$), it makes possible also the derivation of the data k which is significant in examination, and the "gene-expression ratio between alleles", 1+($\alpha$/k), and is thus superior to known competitive PCR. It should be noted that k', $\alpha$' and (1+($\alpha$/k')) derived (from Formula (V) - Formula (VIII)) by reversing A2 and A1, and, B2 and B1 in Formula (I) to Formula (IV) may be used in place of k, $\alpha$, (1+($\alpha$/k)).

$$k' = (A1/A2) \ \ldots \ (I)$$

$$B1 = B2(\alpha' + k') \ \ldots \ (II)$$

$$\alpha' = | \ (B1/B2) - (A1/A2) | \ \ldots \ (III)$$

$$(1 + (\alpha'/k')) = (B1/B2)/(A1/A2) \ \ldots \ (IV)$$

If the signal strength where both of the two alleles are expressed is established as 100, theoretically, the signal strength where only one of the two alleles is expressed is 50, and where both alleles do not express, the signal strength is 0. In the measurement results of gene expression amounts obtained by conventional methods such as competitive PCR, there is a dispersion of around 30% to 50%. However, if there is a dispersion of around 30% to 50%, it is difficult to accurately distinguish between the case where both of the two alleles are expressed and the case where only one of the alleles is expressed, and also between the case where one allele only is expressed and the case where neither of the two alleles is expressed. With the exception of special instances where only one of the two alleles is expressed due to the phenomenon of genomic imprinting, there are yet no reports of statistically significant differences in gene expression between alleles. One reason that, excluding specials instances, there have been no reports of statistically significant differences in gene expression between, alleles is that there has been insufficient accuracy in detection. It should be noted that the dispersion in detection in the method of the present invention is below 10%, several % average (Described below.) The method of the present invention can be applied not only to single nucleotide polymorphisms but to genes having cancer-specific mutations.

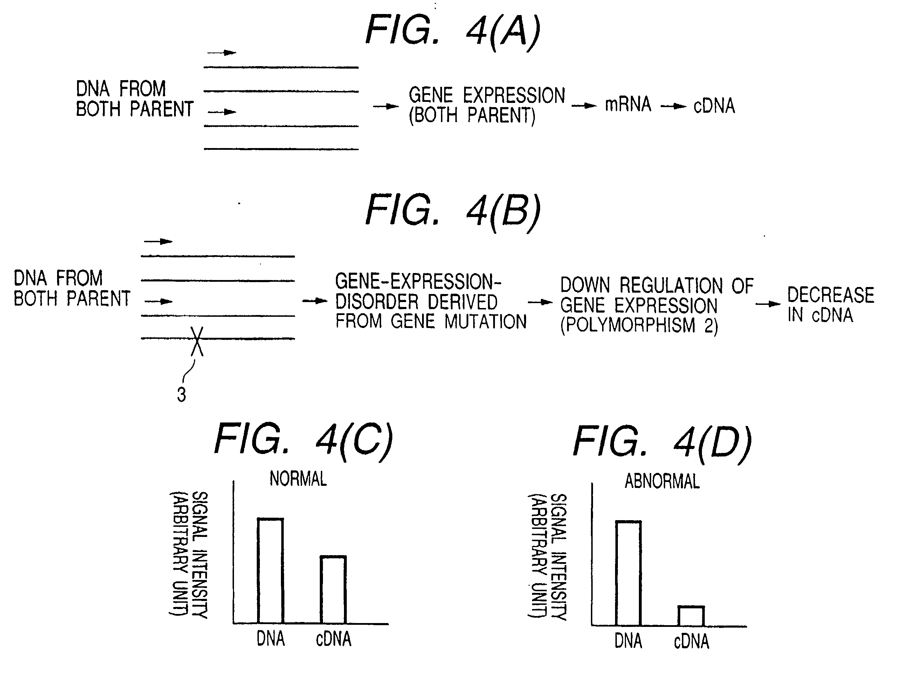

Figure 4 is a figure explaining a screening example suitable for cancer cells having a mutation of one of the alleles, without having a single nucleotide polymorphism. Figure 4 (A) is a figure explaining the relationship between a normal gene and the transcript of the normal gene. Figure 4(B) is a figure explaining the relationship between abnormal gene of a cancer cell or the like, and the transcript of the abnormal gene. Figure 4(C) is a figure indicating the amount of "genomic DNA fragment" and "complementary DNA fragment" present in the case of a normal gene. Figure 4 (D) is a figure indicating the amount of "genomic DNA fragment" and "complementary DNA fragment" present in the case of an abnormal gene. Since in the normal gene there is no single nucleotide polymorphism, the two alleles cannot be distinguished. However, where there is a cancer-specific mutation 3 present in a target region, there will often occur abnormalities in gene expression and a reduction in the amount of mRNA, and thus, a reduction in the amount of cDNA that can be obtained by reverse transcriptase reaction. From the electrophoresis pattern of the "genomic DNA fragment" and the electrophoresis pattern of the "complementary DNA fragment", the ratio of "genomic DNA fragment" to "complementary DNA fragment" will exhibit as significant difference thereby allowing the significance of the mutation to be established. In this embodiment, since analysis is conducted with genomic DNA and RNA collected from the sample screening sample, when compared with using either one of genomic DNA, RNA as a sample, there is no difference in the impact on the screening subject.

The method of electrophoresis is influenced by gel composition, gel freezing point, time etc, however, where as in the present embodiment, the ratios of peak height and peak area of the two alleles are used as indicators, the variance in the result data is very low. An examination using the results of the present embodiment is superior to examination methods using conventional methods in that there are fewer misdiagnoses. Where the "genomic DNA fragment" and the "complementary DNA fragment" are labeled with differing fluorescent labels, and electrophoresed on the same lane, variance between lanes can be reduced allowing more precise measurement.

The genetic screening method of the present invention is applied, and by means of fluorescence-detection type genetic screening apparatus comprising a plurality of electrophoresis lanes for electrophoresis of nucleic acid fragments labeled with a fluorescent marker; a means for irradiating a laser onto the plurality of electrophoresis paths; a means for detecting the fluorescence emitted from the fluorescent labels due to irradiation with a laser; a means for analyzing the electrophoresis pattern of nucleic acid fragments separated by electrophoresis; and a display apparatus for displaying the analysis results, there are preferably displayed on the display apparatus thereof, as either letter(s), numerical value(s), or graph(s), 1 or more of any of the following: the name of the target gene site, the nucleotide sequence of the primer, the nucleotide sequences of the alleles (allele 1 and allele 1'), the difference in nucleotide sequence between the alleles (allele 1 and allele 1'), the signal intensity of the electrophoretic band of the "genomic DNA fragment" derived from each of the alleles respectively (allele 1 and allele 1'), the signal intensity of the electrophoretic band of the "complementary DNA fragment" derived from each of the alleles respectively (allele 1 and allele 1'), the ratio of signal intensities of the electrophoretic bands of the "genomic DNA fragments" derived from each of the alleles respectively (allele 1 and allele 1') (i.e. the ratio of the signal intensity of the electrophoretic band of the "genomic DNA fragment" derived from allele 1, to the signal intensity of the electrophoretic band of the "genomic DNA fragment" derived from allele 1'), the ratio of signal intensities of the electrophoretic bands of the "complementary DNA fragments" derived from each of the alleles respectively (allele 1 and allele 1') (i.e. the ratio of the signal intensity of the electrophoretic band of the "complementary DNA fragment" derived from allele 1, to the signal intensity of the electrophoretic band of the "complementary DNA. fragment" derived from allele 1'), and the statistically significant difference of the gene expression ratio/difference between alleles (e.g. difference in gene expression between alleles": ($\alpha$ (Formula III)), "gene-expression-ratio: $(1+(\alpha/k)$, (Formula IV)). The peak height and peak area of the electrophoretic band of the DNA fragment may be used as the signal intensity.

**Examples**

Hereinafter, the present invention will be described in more detail by experimental examples.

1. Isolation of DNA sample

1.1 Isolation of DNA sample from blood

DNA sample was isolated by the following procedures:

(1) To 5 mL of whole blood from a subject was added 0.5% NaCl in water, and erythrocytes were burst in the whole blood under low osmotic pressure.
(2) The solution from (1) was centrifuged to remove erythrocytes and obtain leukocytes.
(3) To the resulting leukocytes was 5 mL of a lysis buffer (10 mM Tris, 0.01 mM EDTA, 0.5% SDS, and 100 $\mu$g/mL RNase, all in final concentration), and the buffer was maintained at a temperature of 37°C for 1 hour.
(4) Protease K (final concentration, 50 $\mu$g/mL) was added to the lysis buffer, followed by overnight reaction at 55°C.
(5) To the solution from (4) was an equal volume of saturated phenol, and the mixture was shaken at room temperature and centrifuged at 3,000 rpm for 10 min.
(6) Following centrifugation, the upper layer was recovered.
(7) Phenol-chloroform was then added in a volume equal to that of the upper layer of (6), and the mixture was shaken at room temperature and centrifuged at 3,000 rpm for 10 min. to recover upper layer.
(8) Chloroform was added in a volume equal to that of the upper layer of (7), and the mixture was shaken at room temperature and centrifuged at 3,000 rpm for 10 min. to recover upper layer.
(9) The upper layer, recovered in (8), was subjected to ethanol precipitation to obtain DNA.

1.2 Isolation of DNA sample from tissue

After removal of a tissue, the tissue was rapidly frozen with liquid N$_2$ and stored. It was destroyed upon use, and DNA was obtained in accordance with the procedures (3)-(9) of 1.1 above.

2. Isolation of RNA

RNA was isolated by the following procedures:

(1) To 5 mL of whole blood from a subject was added 0.5% NaCl in water, and erythrocytes were burst in the whole blood under low osmotic pressure.
(2) Erythrocytes were removed from the solution of (1) to obtain leukocytes.
(3) 500 $\mu$L of cytolytic solution D (4 M guanidium thiocyanate, 25 mM sodium citrate (pH 7.0), 0.5% sarcosyl, and 0.1 M 2-mercaptoethanol) was added per 50 mg tissue and mixed.
(4) 50 $\mu$L of 2 M sodium acetate (pH 4.0) was added to the solution of (3) and mixed, and the mixture was repeatedly centrifuged.
(5) To the solution from (4) were added RNA-free saturated phenol 500 $\mu$L and chloroform/isoamyl alcohol (49:1) 100 $\mu$L.
(6) Following mixing, the mixture was left on ice for 15 min.
(7) The solution from (6) was centrifuged at 10,000 rpm at 4°C for 20 min.
(8) Upper layer was collected.
(9) Ethanol 1 mL was added to the upper layer collected in (8) for precipitation at 20°C for 1 hr.
(10) The solution from (9) was centrifuged at 10,000 rpm at 4°C for 20 min. -
(11) The upper layer was decanted, and the resulting precipitate was lysed in 300 $\mu$L cytolytic solution D.
(12) Ethanol 1 mL was added to the lysate solution of (11) for reprecipitation at 20°C for 1 hr.
(13) The solution from (12) was centrifuged at 10,000 rpm at 4°C for 20 min.
(14) After decant of the upper layer, the precipitate was washed with 75% ice-cold ethanol and centrifuged for 5 min.
(15) 0.1% diethyl pyrocarbonate solution 50 $\mu$L was added to the precipitate of (14) to dissolve RNA.
(16) The dissolved RNA was stored at - 70°C.

2.2 Isolation of RNA from tissue

After removal of a tissue, the tissue was rapidly frozen with liquid N$_2$ and stored. It was destroyed by homogenization upon use, and RNA was isolated in accordance with the procedures (3) - (16) of 2.1 above.

3. Reverse transcriptase (RT) reaction

RT reaction was conducted as follows.

(1) The following (1A)-(1D) were mixed to prepare a mix solution (total 10 $\mu$l) :

(1A), RNA solution (1 $\mu$g/$\mu$L) 1.0 $\mu$L;
(1B), RT primer (50 nM) 1.0 $\mu$L;
(1C), 10 x reverse transcriptase buffer 1.4 $\mu$L;
(1D), ion-exchanged water 6.6 $\mu$L.

(2) The mix solution was heated at 95°C for 2 min.
(3) The mix solution was subjected to one-hour reaction at 55°C.
(4) The resulting mixture was centrifuged.
(5) The following (5A)-(5E) were mixed to prepare a mix solution (total 14.0 $\mu$L) :

(5A), the mixture 1 of (4) 10.0 $\mu$L;
(5B), dithiothreitol (100 mM) 1.4 $\mu$L;
(5C), dNTPs (where N=A, T, G or C) mix (5 mM each) 1.4 $\mu$L;
(5D), RNasin (40 U/$\mu$L, Gibco BRL) 0.7 $\mu$L;
(5E), reverse transcriptase Superscript II (200 U/$\mu$L, Gibco BRL) .

(6) The mix solution was kept at 37°C for 1 hour.

(7) The mix solution was maintained at 80°C for 5 min.
(8) The resulting mixture 2 was centrifuged and left on ice.

4. PCR

PCR was conducted as follows.

(1) The following (1A)-(1H) were mixed to prepare a mix solution (total 10 $\mu$L) :

(1A) genomic DNA or cDNA (0.5 $\mu$g/ $\mu$L) 1 $\mu$L;
(1B), PCR primer (forward, 10 nmol/mL) 0.25 $\mu$L;
(1C), fluorescence-labeled PCR primer (reverse, 10 nmol/mL) 0.25 $\mu$L ;
(1D), MgCl$_2$ (25 mM) 0.4 $\mu$L;
(1E), dNTPs (where N=A, T, G or C) mix (2.5 $\mu$mol/mL) 0.8 $\mu$L;
(1F), Taq polymerase (5 U/ $\mu$L) 0.05 $\mu$L;
(1G), 10 x PCR buffer 1 $\mu$L;
(1H), redistilled water 6.25 $\mu$L,

where used as the fluorescent label for primer was Cy 5 (Amersham Pharmacia).
(2) The mix solution was subjected to PCR amplification under conditions as exemplified below:

denaturation, 94°C for 5 min.;
30 PCR cycles of 94°C for 30 sec., 60°C for 30 sec., and 72°C for 1 min., whereby gene was amplified; and
extension reaction, 72°C for 8 min.

By these procedures, PCR amplification product (total 10 $\mu$L) was obtained.

5. Blunting treatment

To the PCR amplification product 10 $\mu$L was added 1. 0 U Klenow fragment, and the reaction was conducted at 37°C for 30 min.

6. Detection of single-strand conformation polymorphism (SSCP)

(1) To the blunted PCR amplification product was added a formamide staining solution (90% formamide, 20 mM EDTA, 0.05% bromophenol blue) in a 5-10 fold volume.
(2) For heat denaturation, the solution from (1) was heated at 90°C for 5 min.
(3) The PCR amplification product was separated by electrophoresis on non-denatured 15% acrylamide gel, using a fluorescence detection type DNA sequencer (ALF Express, Amersham Pharmacia). Electrophoresis buffer employed was Tris-glycine buffer (25 mM Tris, 192 mM glycine). The electrophoresis was run at 20°C. at a fixed electric power 30W for 6 hours. "Genomic DNA fragment" and "cDNA fragment" were electrophoresed concurrently on the same gel. Fluorescence, which was emitted by exciting the fluorescent label with laser, was detected by a photo sensor, and the relation of time after the beginning of electrophoresis (electrophoresis time) with quantity of light detected by photo sensor was recorded.

7. Comparison of electropherograms between "genomic DNA fragment" and "cDNA fragment"

Fig. 5 depicts an example of the electropherograms determined by the method for screening genes according to the present invention. Figs. 5(A) and 5(B) show the electropherograms of normal and abnormal samples, respectively. In the example of Fig. 5 the target was a single nucleotide polymorphism of the exon 11 of BRCA 1 gene, which gene is expressed systemically, for example in blood, and is capable of preparing from DNA or RNA that is extracted from leukocytes of a patient with familial breast cancer. In Figs. 5(A) and 5(B), the transverse axis represents electrophoresis time, and the vertical axis represents a quantity of fluorescent light from fluorescence-labeled primer (in counts). Area of electrophoretic band 41 of "genomic DNA fragment" from allele 1 is represented as S1(DNA), and peak value of the band as P1(DNA); area of electrophoretic band 42 of "genomic DNA fragment from allele 1' is represented as S2 (DNA), and peak value of the band as P2 (DNA) ; area of electrophoretic band 43 of "cDNA fragment" from allele 1 is represented as S1 (cDNA), and peak value of the band as P1 (cDNA) ; and area of electrophoretic band 44 of "cDNA fragment" from allele 1' is represented as S2 (cDNA), and peak value of the band as P2(cDNA). The area and peak value of each DNA band were determined using a Peak Detection function set to default in an analytical software (Allele Link) attached to ALF Express. In the normal sample in Fig. 5(A), S2 (DNA) /S1 (DNA) is consistent with S2 (cDNA) /S1 (cDNA), with an error within the range of $\pm$ 8%, whereas in the abnormal sample in Fig. 5 (B) where it is supposed that there is a difference in gene expression between alleles, the both did not coincide evidently with each other. From the results of Figs. 5(A) and 5(B), similar results were seen in comparison of P2 (DNA) /P1(DNA) with P2 (cDNA)/P1 (cDNA) . In the example in Fig. 5 (B) it was suggested that there exists a difference in gene expression between the alleles, as seen in the example of Fig. 3. Primers used to obtain Figs. 5 (A) and 5 (B) are shown in SEQ ID NOS:1 and 2, which can amplify part of the exon 11 region of BRCA1 gene. Similar results could also be obtained by use of primers of SEQ ID NOS:3 and 4 etc. , which are capable of amplifying another region of the exon 11 of BRCA1 gene. SEQ ID NOS:1 to 4 are as follows.

TTGTCAATCCTAGCCTTCCAAGAG (SEQ ID NO:1)

TTTTGCCTTCCCTAGAGTGCTAAC (SEQ ID NO:2)

GCAACTGGAGCCAAGAAGAGTAAC (SEQ ID NO:3)

TTTGCAAAACCCTTTCTCCACTTA (SEQ ID NO:4)

8. Effect of PCR conditions on results

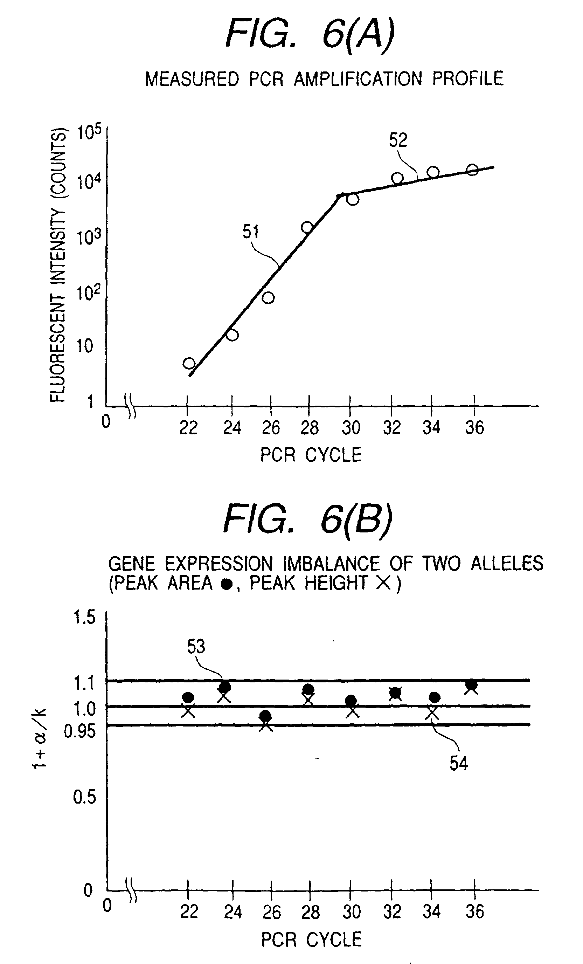

Fig. 6 shows an effect of PCR cycle number in the method for screening genes according to the present invention, where Fig. 6(A) shows the relation of a PCR cycle number and a DNA copy number, and Fig. 6(B) shows the relation of a PCR cycle number and a "gene expression imbalance of two alleles". The target was a single nucleotide polymorphism of the exon 4 of p53 gene, which gene is expressed systemically, for example in blood, and can be prepared from DNA or RNA which is extracted from leukocytes of a healthy person. Primers used in the examples of Figs. 6(A) and 6(B) are represented as SEQ ID NOS:5 and 6:

AGCTCCCAGAATGCCAGAG (SEQ ID NO:5);

and

CTGGGAAGGGACAGAAGATG (SEQ ID NO:6).

In the examples shown in Figs. 6(A) and 6 (B), a sample from healthy person (i.e., DNA, cDNA) was employed as a template, and it was denatured at 94°C for 5 min. and was subsequently subjected to PCR amplification (94°C for 30 sec., 60°C for 30 sec., and 72°C for 1 min.) of 22, 24, 26, 28, 30, 32, 34 and 36 cycles. In Fig. 6(A) the transverse axis represents a PCR cycle while the vertical axis is a fluorescence quantity emitted from a fluorescence labeled primer (in counts). Since the DNA copy number is proportional to the fluorescence count, the vertical axis represents a DNA copy number. At 22-28 PCR cycles the exponential amplification phase was 51, and at 30 or more PCR cycles the saturation phase became 52. In Fig. 6(B) a closed circle 53 indicates a value of (S2 (cDNA) /S1 (cDNA)) / (S2 (DNA) /S1 (DNA)), and the symbol x 54 represents a value of (P2(cDNA)/P1(cDNA))/(P2(DNA)/P1(DNA)). In this figure, all the points are in the range from 0.93 to 1.10. For the normal sample, the difference in gene expression between alleles is not so large and the gene expression ratio of two alleles is likely to be nearly 1, thus the results in Fig. 6 (B) are reasonable. While in conventional methods like competitive PCR and kinetic PCR the quantitative profile is ensured only at the exponential amplification phase 51, in the present example, in which the both alleles are compared, reasonable screening results can be obtained even on the saturation phase 52. Thus, if the amount of a DNA or cDNA sample is small, then PCR cycle number can be increased so that sensitive measurement becomes possible even in a small amount of the sample. Furthermore, if cDNA sample differs in its amount from DNA sample, for example if the amount of cDNA sample is 10-fold smaller than that of DNA sample, it is realized from the results in Fig. 6 (B) that the PCR cycle number of cDNA can be set so as to become two or three cycles higher than that of DNA sample. Additionally, the results in Fig. 6(B) indicates that any result obtained by the method for screening genes according to the present invention is almost not affected by PCR cycle number. Thus, the gene expression imbalance of two alleles (i.e. 1+($\alpha$/k), indicated as formula IV) is almost equivalent, with a dispersion below a few % even when PCR cycle number is varied.

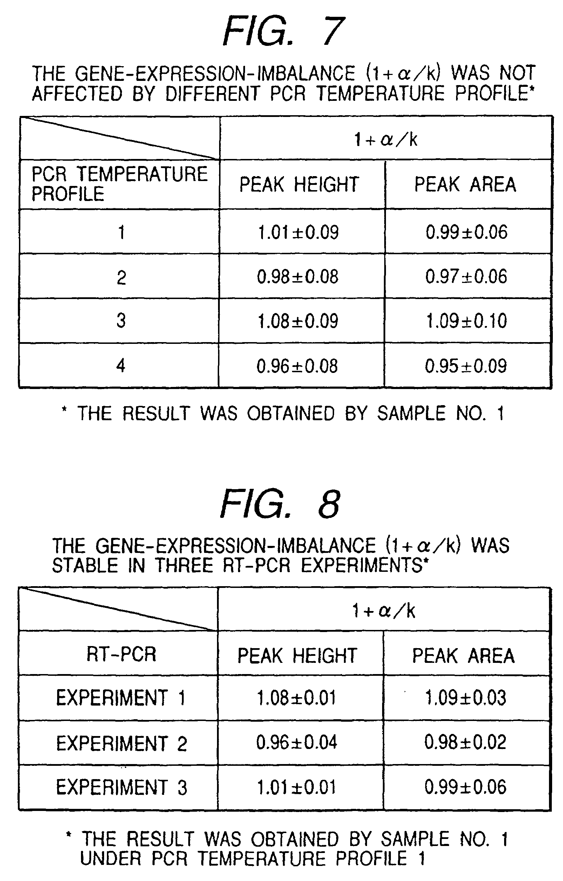

Fig. 7 shows an effect of PCR temperature profile in the method for screening genes according to the present invention. The results shown in this figure were obtained by using the same sample as in Figs. 6(A) and 6(B). The PCR temperature profiles (i.e., combinations of temperature and time in denaturation, annealing and extension) 1 to 4 are as follows.

PCR temperature profile 1: denaturation at 94°C for 5 min.; then 30 PCR cycles of 94°C for 30 sec., 60°C for 30 sec., and 72°C for 1 min. PCR temperature profile 2: denaturation at 94°C for 5 min.; then 30 PCR cycles of 94°C for 30 sec., 60°C for 30 sec., and 72°C for 30 sec.

PCR temperature profile 3: denaturation at 94°C for 5 min.; then 30 PCR cycles of 94°C for 30 sec., 55°C for 30 sec., and 72°C for 1 min. PCR temperature profile 4: denaturation at 94°C for 5 min.; then 30 PCR cycles of 94°C for 30 sec., 55°C for 30 sec., and 72°C for 30 sec.

In Fig. 7, the ratio of gene expression between alleles (i.e. 1+($\alpha$/k), indicated as formula IV) is represented by H2/H1 = (S2(cDNA)/S1(cDNA))/ (S2(DNA)/S1(DNA)) and A2/A1 = (P2(cDNA)/P1(cDNA))/ (P2(DNA)/P1(DNA)). Fig. 7 further shows average (1+$\alpha$/k) values and standard deviations (which are numerals following $\pm$), which were determined by multiple measurements. In PCR temperature profiles 1 to 4, (S2(cDNA)/S1(cDNA))/ (S2(DNA)/S1(DNA)) was in the range from 0.96 to 1.08, and (P2(cDNA)/P1(cDNA))/ (P2(DNA)/P1(DNA)) was 0.95 to 1.09. In addition, dispersions of (1+$\alpha$/k) values in PCR temperature profiles 1 to 4 were 6.1%, 6.2%, 9.2% and 9.5%, respectively, as determined using each area, and the maximum dispersion from the (1+$\alpha$/k) value = 1, was 9%. The results in Fig. 7 indicates that results obtained by the method of this invention were almost not affected by PCR temperature profiles under conditions where DNA or cDNA as template is amplified; that is, said dispersion was up to 10%.

9. Dispersion of results determined between RT-PCRs and between samples

Fig. 8 shows dispersion of three results individually obtained by RT-PCR in the method for screening genes according to the present invention. In each RT-PCR shown in Fig. 8, reverse transcriptase (Superscript II reverse transcriptase) from different tubes was employed and the target was a single nucleotide polymorphism of the exon 4 of p53 gene. The primers used were ones shown in SEQ ID NOS:5 and 6, which amplify a region of the exon 4 of p53 gene. In the example shown in Fig. 8, PCR temperature profile 1 was employed. Further, the ratio of gene expression between alleles (i. e. 1+ ($\alpha$/k), indicated as formula IV) was represented in the same manner as in Fig. 7. The sample used in Fig. 8 was identical to that in Fig. 7. As seen in Fig. 8, dispersions of the results individually obtained in runs 1, 2 and 3 were 2.8%, 2.0% and 6.1% respectively, as determined using area, which values were several % or less. The maximum dispersion from (1+$\alpha$/k) = 1, was 9%. The dispersion not more than several %, which was obtained by respective runs using the same sample, was smaller than the maximum dispersion 10% which is due to a difference between PCR temperature profiles.

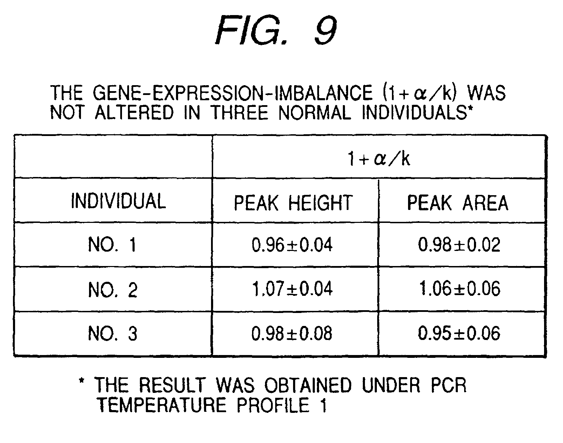

Fig. 9 shows dispersions of results obtained for three different samples by the method for screening genes according to the present invention. The ratio of gene expression between alleles, (1+$\alpha$/k) of formula IV, was represented in the same manner as in Fig. 7. The sample used in Figs. 6, 7 and 8 was identical to sample No. 1 in Fig. 9. Used as PCR temperature profile was its No. 1. As seen in Fig. 9, dispersions of the results individually obtained in sample Nos. 1, 2 and 3 were 2.0%, 5.7% and 6.3%, respectively, as determined using area, each of which was several % or less, indicating that all results obtained were reasonable. As shown in Figs. 6 to 9, the dispersion was as low as several % or less under the same PCR temperature profile conditions in the case of the method of this invention.

10. Display example of screening results

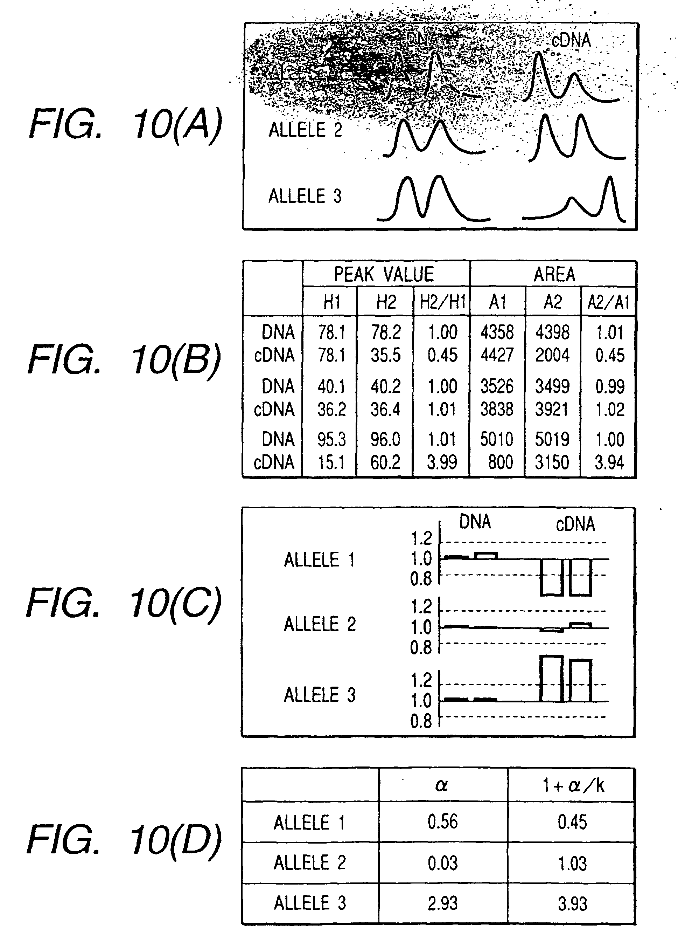

Fig. 10 is a display example that represents screening results obtained by the method of this invention upon medical examination of individuals. Fig. 10(A) is a display example representing an electrophoretic pattern (or electropherogram) for each locus of an individual tested, wherein the transverse axis is electrophoresis time and the vertical axis is fluorescent intensity. Fig. 10 (B) is a display example that represents peak values of electrophoretic bands (H1, H2), calculated areas (A1, A2), and ratios (H2/H1, A2/A1), by numerals. These values were determined by analyzing the electrophoretic bands on the electrophoresis pattern of each locus shown in Fig. 10 (A). In the display example of Fig. 10(B) from the left row of the first line in the display toward the right row of the same line, the locus 1 is displayed in the order of: H1 = P1 (DNA) ; H2 = P2 (DNA) ; H2/H1 = P2(DNA)/P1(DNA); A1 = S1 (DNA) ; A2 = S2 (DNA) ; and A2/A1 = S2 (DNA) /S1 (DNA) ; and from the left row of the second line in the display toward the right row of the same line, the locus 1 is displayed in the order of: H1 = P1(cDNA); H2 = P2(cDNA); H2/H1 = P2(cDNA)/P1(cDNA); A1 = S1(cDNA); A2 = S2(cDNA); and A2/A1 = S2(cDNA)/S1(cDNA). Loci 2 and 3 are also displayed in the same manner.

Fig. 10 (C) is a display example that displays H2/H1 = P2 (DNA) /P1 (DNA), A2/A1 = S2(DNA)/S1(DNA), H2/H1 = P2(cDNA)/P1(cDNA), and A2/A1 S2(cDNA)/S1(cDNA) for each locus, by a histogram. Where gene expression is normal, each of the above four ratios is ideally 1.0. In this figure the vertical axis represents any of the four ratios. Based on results obtained by for example a group medical examination, if it is previously known that in healthy people each ratio is in the range from 0. 8 to 1.2 then the boundary between normal and abnormal is represented by dot lines. In the loci 1 and 3 of Fig. 10 (C), because bars exceed the boundary, abnormal gene expression is apparently suggested in loci 1 and 3. In Fig. 10(D), difference in gene expression between alleles ($\alpha$, as formula III) and gene expression imbalance of two alleles (1+($\alpha$/k) as formula IV) are presented for each locus by numerals.

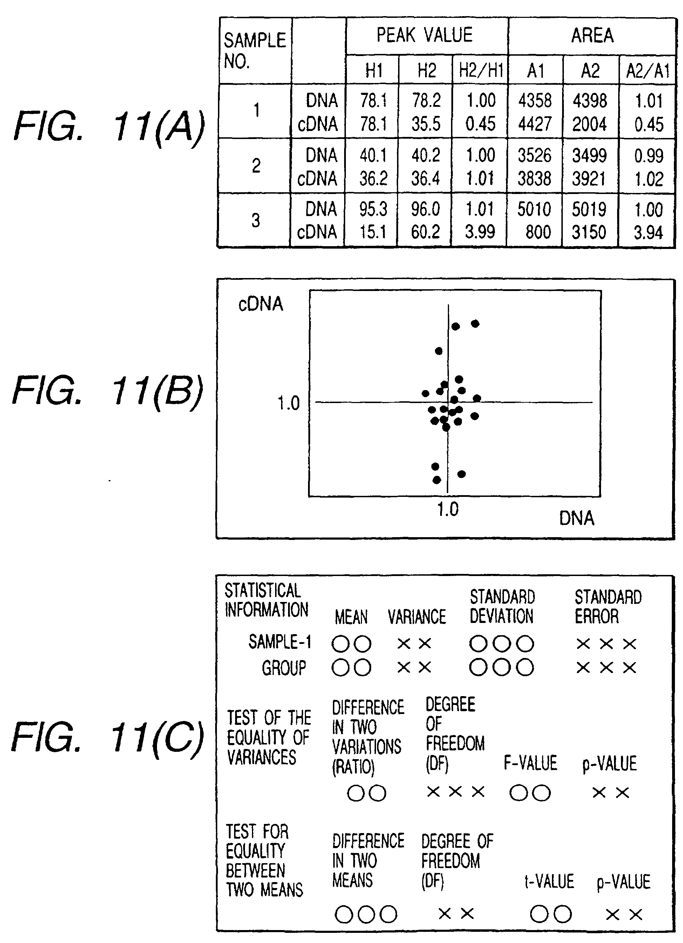

Fig. 11 is a display example that displays screening results obtained upon group medical examination by the method of the present invention. The display in Fig. 11(A) is similar to that in Fig. 10(B) and is based on screening results for a certain locus in a plurality of subjects (or samples) . In Fig. 11 (B), the transverse axis represents H2/H1 = P2 (DNA) /P1 (DNA) or A2/A1 = S2 (DNA) /S1 (DNA), and the vertical axis represents H2/H1 = P2(cDNA)/P1(cDNA) or A2/A1 = S2 (cDNA) /S1 (cDNA) . These ratios that were determined by screening each sample are represented by dots. If gene expression is normal, ideally the coordinate of each dot is (1.0, 1.0) . Many dots obtained by this screening are present near (1.0, 1.0) . If there is a sample with abnormality of gene expression among samples to be tested, dots for sample having such abnormality are displayed at positions apart from (1.0, 1.0), whereby one can readily identify the abnormal samples. For example, when clicking such a dot with a pointing apparatus like mouse, one can refer to more detailed screening results regarding an abnormal sample.

Fig. 11(C) is a display example showing a statistical significance between an abnormal sample, which is displayed at a position apart from (1.0, 1.0) and is designated by clicking it with a pointing apparatus, and a population including no abnormal sample, the statistical significance being calculated by for example t-test (i.e., test for equality between two mean values) or F-test (i.e., test of the equality of variances). Thus this figure is a display example indicating results from statistical treatment of a lot of tested samples. Displayed are a mean value, a variance, a standard deviation and a standard error with respect to abnormal samples and a population. Additionally, displayed for t-test are a difference in two mean values, a ratio of two variations, a degree of freedom (DF), a t-value, and a p-value, while for F-test, a ratio of two variations, a DF, a F-value and a p-value. Any one of t-test and F-test may be displayed with respect to its results.

11. Example of applying probe hybridization to the method for screening genes according to the present invention

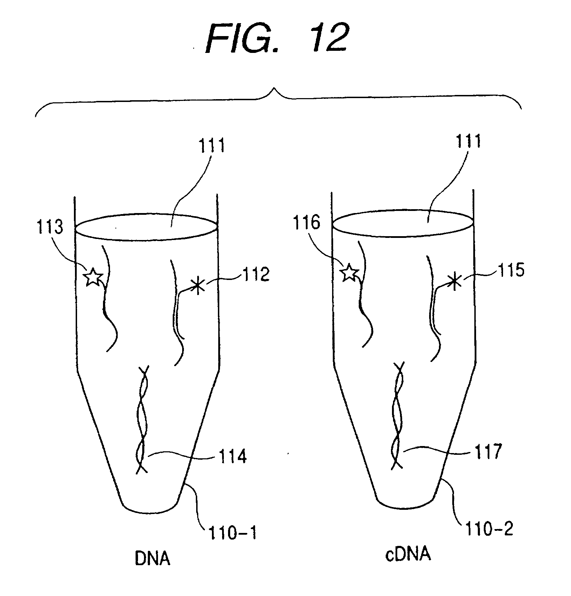

Fig. 12 shows an example of applying DNA-DNA hybridization to the method for screening genes according to the present invention. First, genomic DNA and cDNA are amplified in tubes 110-1 and 110-2 containing a PCR buffer. For example, the amplification of genomic DNA and cDNA is performed in tubes 110-1 and 110-2, respectively. Then, amplified "genomic DNA fragment" 114 and "cDNA fragment" 117 are separately denatured to a single strand by heat treatment or the like. Following denaturation, to tube 110-1 are added a DNA probe 112, which hybridizes specifically with allele 1 of genomic DNA, and a DNA probe 113, which hybridizes specifically with allele 1' of genomic DNA. The DNA probes, 112 and 113, each are previously labeled with a fluorescent agent, where in fluorescent agents one has a difference of 10 nm or more in emission wave length when compared to the other. Tube 110-1 is irradiated with light having a wave length which is in the vicinity of an excitation wave length of the fluorescent agent. The generated fluorescence is detected by means of detector. Fluorescent intensities from DNA probes 112 and 113 are corresponding to S1(DNA) and S2 (DNA), respectively. Similarly, to tube 110-2 are added a DNA probe 115, which hybridizes specifically with allele 1 of cDNA, and a DNA probe 116, which hybridizes specifically with allele 1' of cDNA. The DNA probes, 115 and 116, each are previously labeled with a fluorescent agent, where in fluorescent agents one has a difference of 10 nm or more in emission wave length when compared to the other. Tube 110-2 is irradiated with light having a wave length which is in the vicinity of an excitation wave length of the fluorescent agent. The generated fluorescence is detected by means of detector. Fluorescent intensities from DNA probes 115 and 116 correspond to S1(cDNA) and S2(cDNA) respectively, so that S2(DNA)/S1(DNA) and S2(cDNA)/S1(cDNA) can be compared. Alternatively, the method of this invention can also be carried out by probe hybridization.

As described above, the method for screening genes according to the present invention is based on a new point of view, which makes it possible to detect the presence or absence of an abnormal gene or to clarify a significance of nucleotide polymorphism, by utilizing as an indicator a "difference in gene expression between alleles" or a "ratio of gene expression between alleles". In this method, mRNA from each heterozygous allele is separated and a difference in quantity between mRNAs is measured by RT-PCR-SSCP, utilizing nucleotide sequence polymorphism in an exon region of genomic DNA. Furthermore, in this method the heterozygosity of DNA is examined using the same process and is compared to the quantitative difference of mRNA. When the heterozygosity of DNA and the quantitative difference of mRNA are significantly different from each other, it is determined there is an abnormality. Since the ratio of areas or peak heights in electrophoresis pattern is employed as an indicator for comparison, the dispersion of data obtained becomes extremely small, resulting in reduction in misdiagnosis. Additionally, this method makes it possible to diagnose based on differences in gene expression between heterozygous alleles because the dispersion is in the range not more than several % under the same PCR temperature profile conditions.

Advantage of the Invention

The method for screening genes according to the present invention makes it possible to detect the presence or absence of an abnormal gene or to clarify a significance of a genetic abnormality which can not be determined by DNA sequencing only, by utilizing as an indicator a "difference in gene expression between alleles" or a "ratio of gene expression between alleles". Moreover, since the dispersion caused by sample preparation and PCR amplification becomes small, this method is superior to conventional methods in respect of quantification and reproducibility whereby the method or apparatus for screening genes, which is suitable for being automated, can be provided.

**Claims**

1. A method for analyzing nucleic acid, comprising:

   a first step of obtaining genomic DNA fragments and RNA fragments from a sample taken from a subject:

a second step of obtaining complementary DNA fragments to said RNA fragments by a reverse-transcriptase reaction;

a third step of performing PCR amplification using said genomic DNA fragments and said complementary DNA fragments as templates to obtain a first PCR amplification product derived from the target region of said genomic DNA fragments and a second PCR amplification product derived from a target region of said complementary DNA fragments;

a fourth step of measuring an amount of said first PCR amplification product and an amount of said second PCR amplification product in each of a pair of paternally-derived and maternally-derived alleles from which said genomic DNA fragments and said complementary DNA fragments are derived;

a fifth step of determining a first ratio of said amount of said first PCR amplification product of one of said alleles over said amount of said first PCR amplification product of the other of said alleles, and a second ratio of said amount of said second PCR amplification product of one of said alleles over said amount of said second PCR amplification product of the other of said alleles; and

a sixth step of determining the presence or absence of genetic abnormality based on a third ratio of said first ratio to said second ratio or a difference between said first ratio and said second ratio.

2. The method according to claim 1, which further comprises a step of blunting the termini of said first PCR amplification product and said second PCR amplification product.

3. The method according to claim 1, wherein said PCR amplification is made in identical conditions in respect to the templates of both said genomic DNA fragments and said complementary DNA fragments.

4. The method according to claim 1, wherein said fourth step is conducted by measuring the amount of said first PCR amplification product and said second PCR amplification product for each allele which is detected by a single strand conformation polymorphism method.

5. The method according to claim 1, wherein in the PCR amplification reaction in said third step a fluorescence labeled primer is used, said first PCR amplification product and said second PCR amplification product are subjected to electrophoresis, and the measurement in said fourth step is conducted by detecting fluorescence from said fluorescent label.

6. The method according to claim 5, wherein the measurement in said fourth step is conducted based on a first indicator represented by at least one of S1(DNA)/S2(DNA) and S2(DNA)/S1(DNA), wherein S1(DNA) and S2(DNA) respectively represent a peak area of signal intensity of an electrophoretic band of said first PCR amplification product for each said allele from which said genomic DNA fragments are derived; and

at least one of S1(cDNA)/S2(cDNA) and S2(cDNA)/S1(cDNA), wherein S1(cDNA) and S2(cDNA) respectively represent a peak area of signal intensity of an electrophoretic band of said second PCR amplification product for each said allele from which said complementary DNA fragments are derived,

wherein the difference in gene expression between alleles is detected by a comparison of said first indicator and said second indicator in said fifth step.

7. The method according to claim 5, wherein the measurement in said fourth step is conducted based on a first indicator represented by at least one of P1(DNA)/P2(DNA) and P1(cDNA)/P2(cDNA), wherein the P1(DNA) and the P2(DNA) respectively represent a peak height of signal intensity of an electrophoretic band of said first PCR amplification product for each of the alleles from which said genomic DNA fragments are derived; and

a second indicator represented by at least one of P1(cDNA)/P2(cDNA) and P2(cDNA)/P1(cDNA), wherein P1(cDNA) and P2(cDNA) respectively represent a peak height of signal intensity of an electrophoretic band of said second PCR amplification product for each of the alleles from which said complementary DNA fragments are derived,

wherein the difference in gene expression between alleles is detected by a comparison of said first indicator and said second indicator in said fifth step.

8. The method according to claim 6 further comprising a step of displaying said first indicator and said second indicator numerically or graphically.

**Patentansprüche**

1. Verfahren zum Analysieren von Nucleinsäuren, umfassend:

einen ersten Schritt des Gewinnens von genomischen DNA-Fragmenten und RNA-Fragmenten aus einer aus einem Subjekt entnommenen Probe;

einen zweiten Schritt des Gewinnens von zu den RNA-Fragmenten komplementären DNA-Fragmenten mittels einer Reverse-Transcriptase-Reaktion;

einen dritten Schritt des Durchführens einer PCR-Amplifikation unter Verwendung dieser genomischen DNA-Fragmente und der komplementären DNA-Fragmente als Matrizen, wobei ein erstes, von dem Zielbereich der genomischen DNA-Fragmente abgeleitetes PCR-Amplifizierungsprodukt und ein zweites, von dem Zielbereich der komplementären DNA-Fragmente abgeleitetes PCR-Amplifizierungsprodukts gewonnen wird;

einen vierten Schritt des Messens einer Menge des ersten PCR-Amplifizierungsprodukts und einer Menge des zweiten PCR-Amplifizierungsprodukts in jeweils einem Paar von vom Vater abgeleiteten und von der Mutter abgeleiteten Allelen, von welchen die genomischen DNA-Fragmente und die komplementären DNA-Fragmente abgeleitet sind;

einen fünften Schritt des Bestimmens eines ersten Verhältnisses der Menge des ersten PCR-Amplifizierungsprodukts eines der Allele gegenüber der Menge des ersten PCR-Amplifizierungsprodukts des anderen der Allele, und eines zweiten Verhältnisses der Menge des zweiten PCR-Amplifizierungsprodukts eines der Allele gegenüber der Menge des zweiten PCR-Amplifizierungsprodukts des anderen der Allele; und

einen sechsten Schritt des Bestimmens der Gegenwart oder Abwesenheit einer genetischen Abnormalität, basierend auf einem dritten Verhältnis des ersten Verhältnisses zu dem zweiten Verhältnis oder einer Differenz zwischen dem ersten Verhältnis und dem zweiten Verhältnis.

2. Verfahren nach Anspruch 1, welches weiterhin einen Schritt des Abstumpfens des ersten PCR-Amplifizierungsprodukts und des zweiten PCR-Amplifizierungsprodukts umfasst.

3. Verfahren nach Anspruch 1, wobei die PCR-Amplifizierung unter identischen Bedingungen bezüglich der Matrizen sowohl der genomischen DNA-Fragmente als auch der komplementären DNA-Fragmente durchgeführt wird.

4. Verfahren nach Anspruch 1, wobei der vierte Schritt durchgeführt wird, indem die Menge des ersten PCR-Amplifizierungsprodukts und des zweiten PCR-Amplifizierungsprodukt für jedes Allel, welches durch ein Einzelstrangkonformationspolymorphismusverfahren nachgewiesen wird, gemessen wird.

5. Verfahren nach Anspruch 1, wobei in der PCR-Amplifizierungsreaktion in dem dritten Schritt ein Fluoreszenz-markierter Primer verwendet wird, das erste PCR-Amplifizierungsprodukt und das zweite PCR-Amplifizierungsprodukt einer Elektrophorese unterzogen werden und die Messungen in dem vierten Schritt durch Detektieren der Fluoreszenz des Fluoreszenzmarkers durchgeführt werden.

6. Verfahren nach Anspruch 5, wobei die Messung in dem vierten Schritt basierend auf einem ersten Indikator durchgeführt wird, der durch mindestens eines von S1(DNA)/S2(DNA) und S2(DNA)/S1(DNA), wobei S1(DNA) und S2(DNA) jeweils eine Peakfläche der Signalintensität einer elektrophoretischen Bande des ersten PCR-Amplifizierungsprodukts für jedes der Allele, aus welchem die genomischen DNA-Fragmente abgleitet sind, darstellen; und mindestens eines von S1(cDNA)/S2(cDNA) und S2(cDNA)/S1(cDNA) dargestellt ist, wobei S1(cDNA) und S2(cDNA) jeweils eine Peakläche der Signalintensität einer elektrophoretischen Bande des zweiten PCR-Amplifzierungsprodukts für jedes der Allele darstellen, aus welchen die komplementären DNA-Fragmente abgeleitet sind, wobei der Unterschied der Genexpression zwischen Allelen durch einen Vergleich des ersten Indikators und des zweiten Indikators in dem fünften Schritt nachgewiesen wird.

7. Verfahren nach Anspruch 5, wobei die Messung in dem vierten Schritt basierend auf einem ersten Indikator, der durch mindestens eines von P1(DNA)/P2(DNA) und P1(cDNA)/P2(cDNA) dargestellt wird, wobei P1(DNA) und P2(DNA) jeweils eine Peakhöhe der Signalintensität einer elektrophoretischen Bande des ersten PCR-Amplifizierungsprodukts für jedes der Allele darstellt, aus welchem die genomischen DNA-Fragmente abgeleitet sind; und einem zweiten Indikator durchgeführt wird, der durch mindestens eines von P1(cDNA)/P2(cDNA) und P2(cDNA)/P1(cDNA) dargestellt ist, wobei P1(cDNA) und P2(cDNA) jeweils eine Peakhöhe der Signalintensität einer elektrophoretischen Bande des zweiten PCR-Amplifizierungsprodukts für jedes der Allele darstellt, aus welchen die komplementären DNA-Fragmente abgeleitet sind, wobei der Unterschied der Genexpression zwischen Allelen durch einen Vergleich des ersten Indikators und des zweiten Indikators in dem fünften Schritt nachgewiesen wird.

8. Verfahren nach Anspruch 6, welches weiter einen Schritt der numerischen oder graphischen Darstellung des ersten Indikators und des zweiten Indikators umfaßt.

**Revendications**

1. Procédé pour analyser un acide nucléique, comprenant
une première étape d'obtention de fragments d'ADN génomique et de fragments d'ARN à partir d'un échantillon prélevé sur un sujet ;
une deuxième étape d'obtention de fragments d'ADN complémentaires desdits fragments d'ARN par une réaction de transcriptase inverse ;
une troisième étape de mise en oeuvre d'une amplification par PCR en utilisant lesdits fragments d'ADN génomique et lesdits fragments d'ADN complémentaire comme matrices pour obtenir un premier produit d'amplification par PCR dérivé de la région cible desdits fragments d'ADN génomique et un second produit d'amplification par PCR dérivé d'une région cible desdits fragments d'ADN complémentaire ;
une quatrième étape de mesure d'une quantité dudit premier produit d'amplification par PCR et d'une quantité dudit second produit d'amplification par PCR dans chaque allèle d'une paire d'allèles dérivé du père et dérivé de la mère desquels lesdits fragments d'ADN génomique et lesdits fragments d'ADN complémentaire sont dérivés ;
une cinquième étape de détermination d'un premier rapport de ladite quantité dudit premier produit d'amplification par PCR d'un desdits allèles sur ladite quantité dudit premier produit d'amplification par PCR de l'autre desdits allèles et d'un second rapport de ladite quantité dudit second produit d'amplification par PCR d'un desdits allèles sur ladite quantité dudit second produit d'amplification par PCR de l'autre desdits allèles ; et
une sixième étape de détermination de la présence ou de l'absence d'une anomalie génétique basée sur un troisième rapport dudit premier rapport audit second rapport ou une différence entre ledit premier rapport et ledit second rapport.

2. Procédé selon la revendication 1, qui comprend en outre une étape de formation de bouts francs aux extrémités dudit premier produit d'amplification par PCR et dudit second produit d'amplification par PCR.

3. Procédé selon la revendication 1, dans lequel ladite amplification par PCR est réalisée dans des conditions identiques en termes de matrices desdits fragments d'ADN génomique et desdits fragments d'ADN complémentaire.

4. Procédé selon la revendication 1, dans lequel ladite quatrième étape est effectuée en mesurant la quantité dudit premier produit d'amplification par PCR et dudit second produit d'amplification par PCR pour chaque allèle qui est détecté par un procédé de polymorphisme de conformation de simple brin.

5. Procédé selon la revendication 1, dans lequel, dans la réaction d'amplification par PCR dans ladite troisième étape, est utilisée une amorce marquée par fluorescence, ledit premier produit d'amplification par PCR et ledit second produit d'amplification par PCR sont soumis à une électrophorèse, et la mesure dans ladite quatrième étape est effectuée en détectant la fluorescence dudit marqueur fluorescent.

6. Procédé selon la revendication 5, dans lequel la mesure dans ladite quatrième étape est effectuée en se basant sur un premier indicateur représenté par au moins un parmi S1 (ADN) /S2 (ADN) et S2 (ADN) /S1 (ADN), où S1 (ADN) et 52 (ADN) représentent respectivement une aire de pic d'intensité de signal d'une bande électrophorétique dudit premier produit d'amplification par PCR pour chacun desdits allèles desquels lesdits fragments d'ADN génomique sont dérivés ; et
au moins un parmi S1(ADNc)/S2(ADNc) et S2(ADNc)/S1(ADNc), où S1(ADNc) et S2(ADNc) représentent respectivement une aire de pic d'intensité de signal d'une bande électrophorétique dudit second produit d'amplification par PCR pour chacun desdits allèles desquels lesdits fragments d'ADN complémentaire sont dérivés,
la différence d'expression de gène entre les allèles étant détectée par une comparaison dudit premier indicateur et dudit second indicateur dans ladite cinquième étape.

7. Procédé selon la revendication 5, dans lequel la mesure dans la quatrième étape est effectuée en se basant sur un premier indicateur représenté par au moins un parmi P1 (ADN) /P2 (ADN) et P1(ADNc)/P2(ADNc), où P1 (ADN) et P2(ADN) représentent respectivement une hauteur de pic d'intensité de signal d'une bande électrophorétique dudit premier produit d'amplification par PCR pour chacun desdits allèles desquels lesdits fragments d'ADN génomique sont dérivés ; et
un second indicateur représenté par au moins un parmi P1(ADNc)/P2(ADNc) et P2(ADNc)/P1(ADNc), où P1(ADNc) et P2(ADNc) représentent respectivement une hauteur de pic d'intensité de signal d'une bande électrophorétique dudit second produit d'amplification par PCR pour chacun desdits allèles desquels lesdits fragments d'ADN complémentaire sont dérivés,
la différence d'expression de gène entre les allèles étant détectée par une comparaison dudit premier indicateur et dudit second indicateur dans ladite cinquième étape.

8. Procédé selon la revendication 6, comprenant en outre une étape de représentation numérique ou graphique dudit premier indicateur et dudit second indicateur.

# FIG. 1

(STEP a) ISOLATION OF DNA SAMPLE ⟋120

(STEP b) ISOLATION OF RNA SAMPLE ⟋130

(STEP c) REVERSE TRANSCRIPTASE STEP ⟋140

(STEP d) PCR STEP OF DNA AND RNA SAMPLE ⟋150

160

(STEP e) BLUNTING 3'-TERMINAL OF PCR PRODUCTS

(STEP f) DETECTION OF SNPs IN PCR PRODUCTS ⟋170

(STEP g) COMPARISON OF SIGNAL-INTENSITY-RATIOS BETWEEN DNA-TWO-ALLELES AND cDNA-CORRESPONDING-ALLELES ⟋180

190

SIGNIFICANT DIFFERENCE IN SIGNAL-INTENSITY-RATIOS ARE OBSERVED ?

NO

NORMAL (BALANCED) GENE EXPRESSION 200

YES

GENE EXPRESSION IMBALANCE ⟋210

# FIG. 2(A)

PATERNAL DNA ————— A —————— → GENE EXPRESSION → mRNA → cDNA
(POLYMORPHISM 1) ————— T —————— (PATERNAL) (POLYMORPHISM 1) (POLYMORPHISM 1)

MATERNAL DNA ————— G —————— → GENE EXPRESSION → mRNA → cDNA
(POLYMORPHISM 2) ————— C —————— (MATERNAL) (POLYMORPHISM 2) (POLYMORPHISM 2)

DNA (POLYMORPHISM 1) : DNA (POLYMORPHISM 2)=1 : 1 → cDNA (POLYMORPHISM 1) : cDNA (POLYMORPHISM 2)=1 : 1

# FIG. 2(B)

SIGNAL INTENSITY (ARBITRARY UNIT)

DNA POLYMORPHISM 1

DNA POLYMORPHISM 2

cDNA POLYMORPHISM 1

cDNA POLYMORPHISM 2

EP 1 092 782 B1

# FIG. 3(A)

PATERNAL DNA
(POLYMORPHISM 1) — A — → GENE EXPRESSION (PATERNAL) → mRNA (POLYMORPHISM 1) → cDNA (POLYMORPHISM 1)
— T —

MATERNAL DNA
(POLYMORPHISM 2) — G — → GENE EXPRESSION IMBALANCE (MATERNAL) → DOWN REGULATION OF GENE EXPRESSION (POLYMORPHISM 2) → DECREASE IN cDNA (POLYMORPHISM 2)
— C —

DNA (POLYMORPHISM 1) : DNA (POLYMORPHISM 2)=1 : 1 →✕→ cDNA (POLYMORPHISM 1) : cDNA (POLYMORPHISM 2)=1 : 1

# FIG. 3(B)

SIGNAL INTENSITY (ARBITRARY UNIT)

DNA POLYMORPHISM 1

DNA POLYMORPHISM 2

cDNA POLYMORPHISM 1

cDNA POLYMORPHISM 2

# FIG. 4(A)

DNA FROM
BOTH PARENT
→ GENE EXPRESSION
(BOTH PARENT) → mRNA → cDNA

# FIG. 4(B)

DNA FROM
BOTH PARENT → GENE-EXPRESSION-
DISORDER DERIVED
FROM GENE MUTATION → DOWN REGULATION OF
GENE EXPRESSION
(POLYMORPHISM 2) → DECREASE
IN cDNA

3

# FIG. 4(C)

NORMAL

SIGNAL INTENSITY
(ARBITRARY UNIT)

DNA    cDNA

# FIG. 4(D)

ABNORMAL

SIGNAL INTENSITY
(ARBITRARY UNIT)

DNA    cDNA

EP 1 092 782 B1

## FIG. 5(A)

ELECTROPHEROGRAM (NORMAL SAMPLE)

## FIG. 5(B)

ELECTROPHEROGRAM (ABNORMAL SAMPLE)

# FIG. 6(A)

MEASURED PCR AMPLIFICATION PROFILE

# FIG. 6(B)

GENE EXPRESSION IMBALANCE OF TWO ALLELES
(PEAK AREA ●, PEAK HEIGHT ✕)

## FIG. 7

THE GENE-EXPRESSION-IMBALANCE (1+α/k) WAS NOT
AFFECTED BY DIFFERENT PCR TEMPERATURE PROFILE*

| | 1+α/k | |
|---|---|---|
| PCR TEMPERATURE PROFILE | PEAK HEIGHT | PEAK AREA |
| 1 | 1.01±0.09 | 0.99±0.06 |
| 2 | 0.98±0.08 | 0.97±0.06 |
| 3 | 1.08±0.09 | 1.09±0.10 |
| 4 | 0.96±0.08 | 0.95±0.09 |

* THE RESULT WAS OBTAINED BY SAMPLE NO. 1

## FIG. 8

THE GENE-EXPRESSION-IMBALANCE (1+α/k) WAS
STABLE IN THREE RT-PCR EXPERIMENTS*

| | 1+α/k | |
|---|---|---|
| RT-PCR | PEAK HEIGHT | PEAK AREA |
| EXPERIMENT 1 | 1.08±0.01 | 1.09±0.03 |
| EXPERIMENT 2 | 0.96±0.04 | 0.98±0.02 |
| EXPERIMENT 3 | 1.01±0.01 | 0.99±0.06 |

* THE RESULT WAS OBTAINED BY SAMPLE NO. 1
UNDER PCR TEMPERATURE PROFILE 1

# FIG. 9

THE GENE-EXPRESSION-IMBALANCE $(1+\alpha/k)$ WAS
NOT ALTERED IN THREE NORMAL INDIVIDUALS*

| | $1+\alpha/k$ | |
|---|---|---|
| INDIVIDUAL | PEAK HEIGHT | PEAK AREA |
| NO. 1 | $0.96\pm0.04$ | $0.98\pm0.02$ |
| NO. 2 | $1.07\pm0.04$ | $1.06\pm0.06$ |
| NO. 3 | $0.98\pm0.08$ | $0.95\pm0.06$ |

* THE RESULT WAS OBTAINED UNDER PCR
  TEMPERATURE PROFILE 1

FIG. 10(A)

FIG. 10(B)

| | PEAK VALUE | | | AREA | | |
|---|---|---|---|---|---|---|
| | H1 | H2 | H2/H1 | A1 | A2 | A2/A1 |
| DNA | 78.1 | 78.2 | 1.00 | 4358 | 4398 | 1.01 |
| cDNA | 78.1 | 35.5 | 0.45 | 4427 | 2004 | 0.45 |
| DNA | 40.1 | 40.2 | 1.00 | 3526 | 3499 | 0.99 |
| cDNA | 36.2 | 36.4 | 1.01 | 3838 | 3921 | 1.02 |
| DNA | 95.3 | 96.0 | 1.01 | 5010 | 5019 | 1.00 |
| cDNA | 15.1 | 60.2 | 3.99 | 800 | 3150 | 3.94 |

FIG. 10(C)

FIG. 10(D)

| | $\alpha$ | $1+\alpha/k$ |
|---|---|---|
| ALLELE 1 | 0.56 | 0.45 |
| ALLELE 2 | 0.03 | 1.03 |
| ALLELE 3 | 2.93 | 3.93 |

*FIG. 11(A)*

| SAMPLE NO. | | PEAK VALUE | | | AREA | | |
|---|---|---|---|---|---|---|---|
| | | H1 | H2 | H2/H1 | A1 | A2 | A2/A1 |
| 1 | DNA | 78.1 | 78.2 | 1.00 | 4358 | 4398 | 1.01 |
| | cDNA | 78.1 | 35.5 | 0.45 | 4427 | 2004 | 0.45 |
| 2 | DNA | 40.1 | 40.2 | 1.00 | 3526 | 3499 | 0.99 |
| | cDNA | 36.2 | 36.4 | 1.01 | 3838 | 3921 | 1.02 |
| 3 | DNA | 95.3 | 96.0 | 1.01 | 5010 | 5019 | 1.00 |
| | cDNA | 15.1 | 60.2 | 3.99 | 800 | 3150 | 3.94 |

*FIG. 11(B)*

*FIG. 11(C)*

| STATISTICAL INFORMATION | MEAN | VARIANCE | STANDARD DEVIATION | STANDARD ERROR |
|---|---|---|---|---|
| SAMPLE-1 | ○○ | ××  | ○○○ | ×××  |
| GROUP | ○○ | ××  | ○○○ | ×××  |

| TEST OF THE EQUALITY OF VARIANCES | DIFFERENCE IN TWO VARIATIONS (RATIO) | DEGREE OF FREEDOM (DF) | F-VALUE | p-VALUE |
|---|---|---|---|---|
| | ○○ | ×××  | ○○ | ××  |

| TEST FOR EQUALITY BETWEEN TWO MEANS | DIFFERENCE IN TWO MEANS | DEGREE OF FREEDOM (DF) | t-VALUE | p-VALUE |
|---|---|---|---|---|
| | ○○○ | ××  | ○○ | ××  |

# FIG. 12

DNA

cDNA

SEQUENCE LISTING

<110> HITACH,LTD.
National Cancer Center

<120> Genetic Screening Method and Genetic Screening Apparatus

<130> PH-1080

<140>
<141>

<150> JP 294257/1999
<151> 15-OCT-1999

<160> 6

<210> 1
<211> 24
<212> DNA
<213> Artificial Sequence

<220>
<223> DNA primer used for PCR.

<400> 1

```
ttgtcaatcc tagccttcca agag                    24
```

<210> 2
<211> 24
<212> DNA
<213> Artificial Sequence

<220>
<223> DNA primer used for PCR.

<400> 2

```
ttttgccttc cctagagtgc taac                    24
```

<210> 3

<211> 24 -
<212> DNA
<213> Artificial Sequence

<220>
<223> DNA primer used for PCR.

<400> 3

```
        gcaactggag ccaagaagag taac                    24
```

<210> 4
<211> 24
<212> DNA
<213> Artificial Sequence

<220>
<223> DNA primer used for PCR.

<400> 4

```
        tttgcaaaac cctttctcca ctta                    24
```

<210> 5
<211> 19
<212> DNA
<213> Artificial Sequence

<220>
<223> DNA primer used for PCR.

<400> 5

```
        agctcccaga atgccagag                          19
```

<210> 6 -
<211> 20
<212> DNA
<213> Artificial Sequence

<220>
<223> DNA primer used for PCR.

<400> 6

```
        ctgggaaggg acagaagatg                         20
```